# EUROPEAN PATENT APPLICATION

(11) **EP 2 947 155 A1**
(43) Date of publication of application: **25.11.2015**
(21) Application number: 14168834.1
(22) Date of filing: 19.05.2014
(51) Int. Cl.: C12Q 1/68, C12Q 1/02, C12N 15/70

(54) **Methods for detecting arsenic ions**

(71) Applicant: Université de Lausanne, 1011 Lausanne (CH)
(72) Inventor: Merulla, Davide, 1007 Lausanne (CH); Van der Meer, Jan, 1020 Renens (CH)
(74) Representative: reuteler & cie SA

(57) **Abstract**

The invention relates to a biologically based test system for the detection of inorganic and organic pollutants in liquid samples including drinking water samples, comprising a genetically modified bacterium producing a reporter protein upon induction of a regulatory protein in presence of said pollutant, as well as methods for preparing said test system and use thereof in the detection of pollutants in liquid samples.

SEQUENCE LISTING

<110>  UNIVERSITE DE LAUSANNE

<120>  METHODS FOR DETECTING ARSENIC IONS

<130>  P1770EP00

<160>  10

<170>  PatentIn version 3.5

<210>  1
<211>  354
<212>  DNA
<213>  Escherichia coli

<400>  1
atgttgcaac taacaccact tcagttattt aaaaacctgt ccgatgaaac ccgtttgggt      60

atcgtgttgt tgctcaggga gatgggagag ttgtgcgtgt gtgatctttg catggcactg     120

gatcaatcac agcccaaaat atcccgtcat ctggcgatgc tacgggaaag tggaatcctt     180

ctggatcgta aacagggaaa atgggttcac taccgcttat caccgcatat tccttcatgg     240

gctgcccaga ttattgagca ggcctggtta agccaacagg acgacgttca ggtcatcgca     300

cgcaagctgg cttcagttaa ctgctccggt agcagtaagg ctgtctgcat ctaa           354

<210>  2
<211>  354
<212>  DNA
<213>  Escherichia coli

<400>  2
atgtcatttc tgttacccat ccaattgttc aaaattcttg ctgatgaaac ccgtctgggc      60

atcgttttac tgctcagcga actgggagag ttatgcgtct gcgatctctg cactgctctc     120

gaccagtcgc agcccaagat ctcccgccac ctggcattgc tgcgtgaaag cgggctattg     180

ctggaccgca agcaaggtaa gtgggttcat taccgcttat caccgcatat tccagcatgg     240

gcggcgaaaa ttattgatga ggcctggcga tgtgaacagg aaaaggttca ggcgattgtc     300

cgcaacctgg ctcgacaaaa ctgttccggg gacagtaaga acatttgcag ttaa           354

<210>  3
<211>  81
<212>  DNA
<213>  Escherichia coli

<400>  3
cagattaatc atatgcgttt ttggttatgt gttgtttgac ttaatatcag agccgagaga      60

tacttgtttt ctacaaagta a                                                81

<210>  4
<211>  29

<212>  DNA
<213>  Artificial Sequence

<220>
<223>  primer for amplification of  PHIarsR-ABS fragment

<400>  4
gagctctgtt gcaactaaca ccacttcag                                         29


<210>  5
<211>  29
<212>  DNA
<213>  Artificial Sequence

<220>
<223>  primer for amplification of  PHIarsR-ABS fragment

<400>  5
tctttagtta gttagggaat tcactagtg                                         29


<210>  6
<211>  27
<212>  DNA
<213>  Artificial Sequence

<220>
<223>  Primer for amplification of PAA-arsR fragment

<400>  6
aaatctgagc tccaattccg acgtcta                                           27


<210>  7
<211>  24
<212>  DNA
<213>  Artificial Sequence

<220>
<223>  Primer for amplification of PAA-arsR fragment

<400>  7
ctgccaggaa ttggggatcg gaag                                              24


<210>  8
<211>  50
<212>  DNA
<213>  Artificial Sequence

<220>
<223>  primer for amplification of arsRK12

<400>  8
cttggatcca aaggagaggg gaaatgtcat ttctgttacc catccaattg                  50


<210>  9
<211>  32
<212>  DNA
<213>  Artificial Sequence

<220>
<223>  primer for amplification of arsRK12

<400>  9
tctagattaa ctgcaaatgt tcttactgtc cc                                    32


<210>  10
<211>  647
<212>  DNA
<213>  Artificial Sequence

<220>
<223>  Synthetic DNA for pPRK12 construction

<400>  10
ggaagcttgc atgcctgcag gtcgactcta gaggatccaa gctttccaag ttatctcacc      60

taccttaagg taatagtgtg attaatcata tgcgtttttg gttatgtgtt gtttgactta     120

atatcagagc cgagagatac ttgttttcta caaaggagag ggaaatgtca tttctgttac     180

ccatccaatt gttcaaaatt cttgctgatg aaacccgtct gggcatcgtt ttactgctca     240

gcgaactggg agagttatgc gtctgcgatc tctgcactgc tctcgaccag tcgcagccca     300

agatctcccg ccacctggca ttgctgcgtg aaagcgggct attgctggac cgcaagcaag     360

gtaagtgggt tcattaccgc ttatcaccgc atattccagc atgggcggcg aaaattattg     420

atgaggcctg gcgatgtgaa caggaaaagg ttcaggcgat tgtccgcaac ctggctcgac     480

aaaactgttc cggggacagt aagaacattt gcagttaaaa aatttgcctg aattccaagt     540

tatccaccta ccttaaggta atagtgtgat tcatcatatg cgtttttggt tatgtgaatt     600

aatcactagt gaattcccta actaactaaa gattaacttt ataagga                   647

## Description

### Field of the Invention

The present invention relates to methods and tools useful for the detection of pollutants such as arsenic ions in drinking water samples.

### Background of the Invention

Contamination of natural waters with arsenic is a recurring problem in both industrialized and developing countries, and drinking water supplies for large populations can have concentrations much higher than the permissible levels (for most European countries and the United States, 10 µg arsenic per liter; elsewhere, 50 µg arsenic per liter). Systemic and chronic exposure to arsenic is known to lead to vascular diseases, to irritations of the skin and of mucous membranes as well as to dermatitis, keratosis and melanosis. Inorganic arsenic is a human carcinogen and ingestion of inorganic arsenic increases the risk of developing cancer of the bladder, liver, kidney and skin (Abernathy et al,Arsenic exposure and health effects, Elsevier Science Ltd, London, 1999*).*

Arsenic contamination most frequently has a natural origin since arsenic is a common element of minerals in the earth's crust. Weathering of such minerals can be accelerated by microbial activity, specifically in anoxygenic groundwaters with abundant available respirable organic matter, which leads to high concentrations of dissolved inorganic arsenic species. Dominant inorganic anions in solution are arsenite [As(III) in AsO₂⁻] or arsenate [As(V) in AsO₄³⁻]. Sources of arsenic can also be anthropogenic and include the application of organoarsenical pesticides, the combustion of fossil fuels, the use of organoarsenical feed additives, mining activities, the disposal of industrial wastes, and the application of arsenic-containing desiccants and preservatives.

Current laboratory techniques, such as atomic absorption and atomic fluorescence spectrometry, inductively coupled plasma techniques and high-pressure liquid chromatography can accurately measure arsenic in an environmental sample to parts per billion (ppb) concentrations, but are expensive to operate and maintain, and they require fully equipped and specialized laboratories. In contrast, regions that have the most extensive arsenic contamination, such as Vietnam, Bangladesh, India and Cambodia, are also the areas with the least access to such advanced techniques, making the need for in field arsenic detection even more urgent. For this reason, there has been increased interest over the last decade in the development of sensors for the field detection of arsenic.

Colorimetric tests, electrochemical sensors and anodic stripping voltammetric probes have been developed and tested in the field (Rahman et al, 2002, Environ. Sci. Technol, 36: 5385-5394). However, large-scale field testing of the most common colorimetric tests showed that they are not sufficiently reliable in the concentration range below 50 µg arsenic per liter, giving rise to a large number of false-positive and false-negative results. On the basis of this poor performance, new colorimetric tests have been developed and tested in the field, but these remain controversial because of their bulkiness, their use of toxic chemicals (i. e. mercury bromide and zinc), and release of arsine gas during the test. For these reasons various alternative protocols and methods have been proposed.

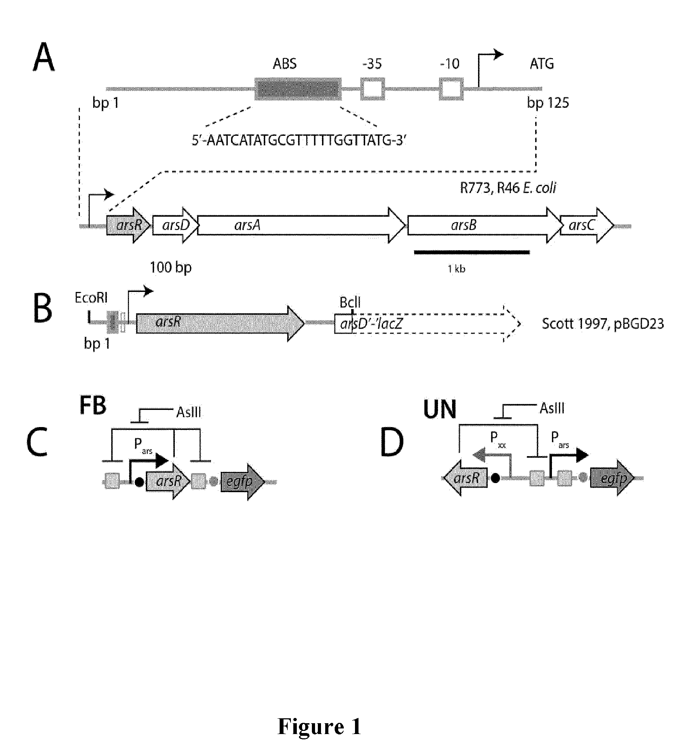

One of the alternatives to the abiotic sensors for arsenic detection is a bacteria-based bioassay (Trang et al, 2005, Environ. Sci. Technol, 39: 7625-7630). The bacteria-based bioassay exploits the extremely sensitive natural defence system of bacteria against arsenite and arsenate, and couples this defence reaction to the production of an easily measurable output. Usually, bacteria-based bioassays or bacterial reporters are genetically modified bacteria comprising a gene coding for a regulatory protein inducing the production of a specific reporter protein (typically a spectroscopically or electrochemically active protein) in the presence of a particular chemicals (e.g. heavy metals such as arsenic, organic compounds). Bacterial reporters for detecting arsenic disclosed so far were typically based on the *arsRDBAC* operon on *E. coli* plasmid R773 (Hedges et al, 1973, J. Bacterial, 115:459-460). The *arsRDBAC* operon is transcribed as a single mRNA and is responsible for the regulation and the production of the proteins of the detoxification system in *E. coli.* ArsR and ArsD are both transacting repressor proteins that homeostatically regulate the levels of *ars* expression (Figure 1A). Although both are 13 kDa protomers and form homodimers, they share no sequence similarity. ArsR is an As(III)/Sb(III)-responsive repressor with high affinity for its operator site (ABS), that controls the basal level of expression of the operon. Binding of arsenite or antimonite leads to dissociation of ArsR from the operator site, permitting transcription to occur more efficiently. ArsD has a lower affinity for the operator site, but it binds at higher protein concentrations. The ArsAB complex is an ATP driven oxyanion efflux pump that removes arsenite and antimonite from the cell. The 583 residues ArsA ATPase is the catalytic subunit whereas ArsB forms the membrane channel. ArsC is a reductase converting pentavalent As (arsenate) into the trivalent form arsenite), which is the substrate of the ArsR regulatory protein and of the efflux pump complex. Typically, it is assumed that the cell cannot avoid entry of arsenite or arsenate. When the cytoplasmic arsenite concentration increases, ArsR releases repression resulting in a rise in the levels of *ars* transcript and consequently an increase in the various Ars gene products. An internal feedback loop exists to avoid that production of the system increases to Inhibitory levels of e.g., the integral membrane protein ArsB. At high levels of ArsD, it acts as a repressor that shuts down *ars* operon expression. Together, ArsR and ArsD form a regulatory circuit that controls both the basal and maximal levels of expression of the *ars* operon.

The first bacterial reporters were developed by fusing a reporter gene downstream of *arsR,* like luciferase or β-galactosidase (Figure 1B) *(*Ramanathan et al, 1997, Anal. Chem. 69: 3380-3384*).* In such a configuration, the production of the reporter gene is dependent on the production of ArsR (coupled system). Because the *arsR* gene is located downstream of its own operator/promoter, the ArsR protein controls its own expression via a negative feedback mechanism. One limitation of this system is the considerable background signal even in the absence of arsenic. To reduce this background signal, a second ABS was inserted between *arsR* and the reporter gene (Figure 1C) in some other bacterial reporters (WO 03102223, Stocker et al., 2003, Environ Sci Technol 37:4743-4750*).*

Recently, feedback-uncoupled arsenic bioreporters were proposed by Merulla et al (microbial Biotechnology, 2013, 5:503-514*),* who explored the effects of uncoupling ArsR from its feedback control (uncoupling the "natural" *Pars-arsR* regulatory element) positioning the ArsR repressor under the control of a constitutive promoter, whereas the reporter gene is maintained under arsenite dependent ArsR/Pars control (Figure 1D). Despite the improvements in the development of bioreporters for arsenic detection, there remains a need for the development and availability of simple, cheap, reliable, accurate test systems, with an improved noise/signal ratio, for the measurement of heavy metals such as arsenic in water samples.

### Summary of the Invention

The present invention is directed towards methods and biologically based test systems for the detection of pollutants in liquid samples such as drinking water.

A first aspect of the invention provides a biologically based test system for the detection of inorganic and organic pollutants in liquid samples comprising a genetically modified bacterium producing a reporter protein upon induction of a regulatory protein in presence of said pollutant.

According to one embodiment of this aspect, the nucleic acid(s) of said bacterium comprises:
(a) a first transcription unit comprising (i) a nucleic acid encoding a reporter protein placed under the control of the promoter from an operon allowing detoxification of said pollutant in bacteria wherein said pollutant is capable of interacting with a regulatory protein of said operon, (ii) a first DNA binding site for said regulatory protein located upstream from said promoter, and (iii) at least a second DNA binding site for said regulatory protein located upstream or downstream from said promoter, and
(b) a second transcription unit comprising (i) a nucleic acid encoding said regulatory protein placed under the control of a constitutive promoter.

In another embodiment of this aspect of the invention, the nucleic acid(s) of said bacterium comprises:
(a) a nucleic acid comprising (i) a nucleic acid encoding a reporter protein placed under the control of the promoter from an operon allowing detoxification of said pollutant in bacteria wherein said pollutant is capable of interacting with a regulatory protein of said operon, in particular the promoter of the chromosomal form of said operon (ii) a first DNA binding site for said regulatory protein located upstream from said promoter, and (iii) at least a second DNA binding site for said regulatory protein located upstream or downstream from said promoter, and
(b) a nucleic acid identical to the chromosomal nucleic acid encoding said regulatory protein.

A second aspect of the invention relates to a recombinant expression vector for use in a biologically based test system for the detection of inorganic and organic pollutants in liquid samples as described herewith.

A third aspect of the invention resides in a host cell such as a bacterium comprising said recombinant expression vector, as well as isolated genetically modified bacteria, for use in a biologically based test system for the detection of inorganic and organic pollutants in liquid samples, producing a reporter protein upon induction of a regulatory protein in presence of said pollutant, as described herewith.

A fourth aspect of the invention concerns a method for preparing said genetically modified bacterium comprising introducing the recombinant expression vector as described herewith in a bacterium.

A fifth aspect relates to a biologically based test kit for the detection of inorganic and organic pollutants in liquid samples as described herewith as well as a method of production of said test kit.

A sixth aspect of the invention provides a device for the detection of inorganic and organic pollutants in liquid samples as described herewith as well as a method of production of said device.

A seventh aspect of the invention provides the use of a biologically based test system as described herewith for the detection of inorganic and organic pollutants in liquid samples.

Other features and advantages of the invention will be apparent from the following detailed description.

### Description of the figures

Figure **1** is a schematic representation of genes and regulatory elements in the design of arsenic bioreporters. (A) Detail of the *ars* promoter region of the *arsRDBAC* operon of plasmid R773 ; (B) Typical *arsR-Pars* based gene circuitry ; (C) Circuitry functioning of an ArsR repressor loop (coupled system) ; (D) Circuitry functioning of an ArsR repressor loop (uncoupled system). Thick arrows: promoters; thin arrows: activation; flat lines: repression.

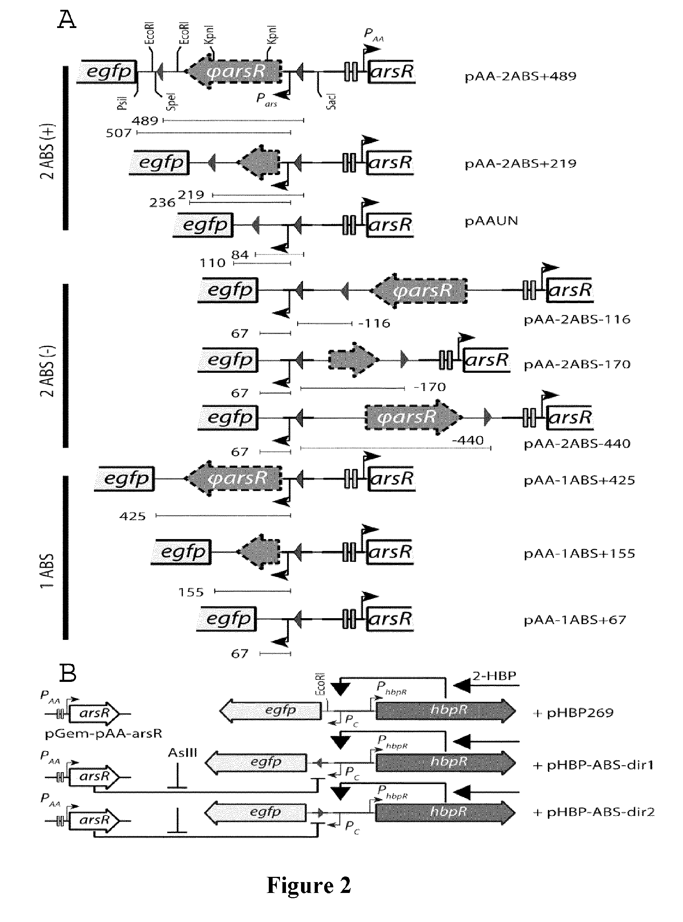

Figure 2 is a shematic representation of the different gene circuits used herewith. (A) Details of the different uncoupled arsenic responsive circuits in which *arsR* is brought under control of the constitutive P_{AA}-promoter, but ArsR maintains control of the Pₐᵣₛ-promoter and its downstream *egfp* reporter gene (*arsR* and *egfp* genes not shown in their entirety). ArsR-binding site (ABS, dark grey triangles) placements, lengths of the corresponding *egfp* 5'UTR and the relative ABS position, distance and orientations are indicated, with relevant plasmid names. 2ABS(+): constructs having a second ABS placed downstream of Pₐᵣₛ. 2ABS(-): constructs having a second ABS upstream of Pₐᵣₛ. 1ABS: constructs with only one ABS in Pₐᵣₛ. All constructs were produced with *arsR* and with an *arsR-mCherry* translational fusion. ϕ*arsR:* frameshifted non-functional *arsR* gene (length of original reading frame stippled). (B) Reporter circuits with heterologous placement of the ArsR ABS within the HbpR-P_{C} system for 2-hydroxybiphenyl (2HBP) detection. These circuits were complemented in *trains* with a plasmid containing *arsR* under the control of P_{AA}. Grey bars within the P_{AA} promoter indicate TetR binding sites.

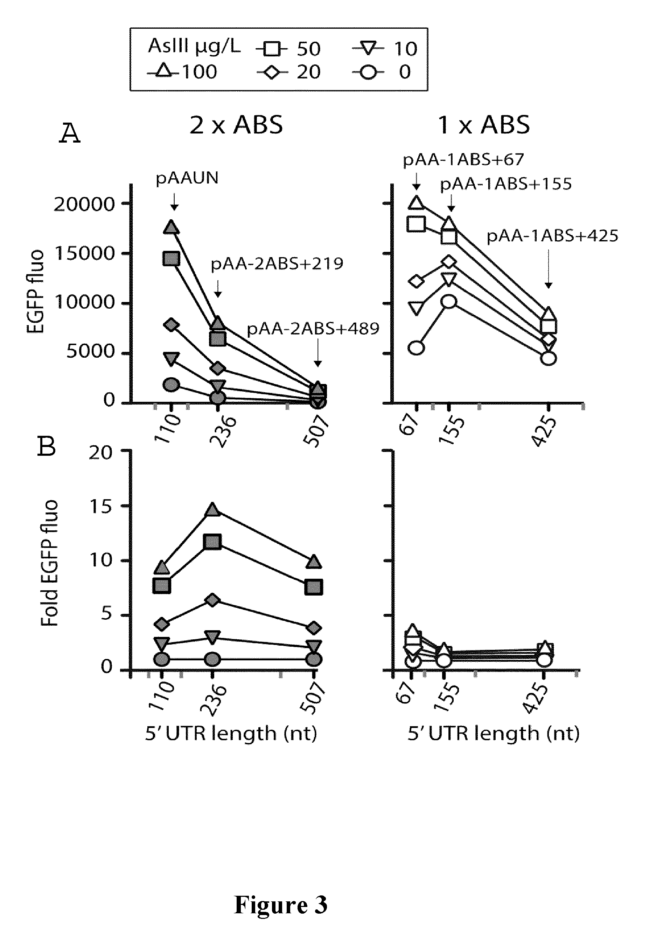

**Figure 3** shows the effect of spacer distance and addition of a second ArsR operator (ABS) on the arsenite inducible EGFP fluorescence in ArsR uncoupled circuits. (A) Mean EGFP fluorescence levels in cells induced or not for 2 h with arsenite (AsIII) (as indicated), as a function of spacer distance (5' UTR length of *egfp* mRNA), measured by flow cytometry (arbitrary units). Relevant plasmid names are indicated above the data points. (B) As for panel (A), but expressed as the ratio between the mean induced EGFP fluorescence and the fluorescence of the cells not exposed to arsenite (Fold EGFP).

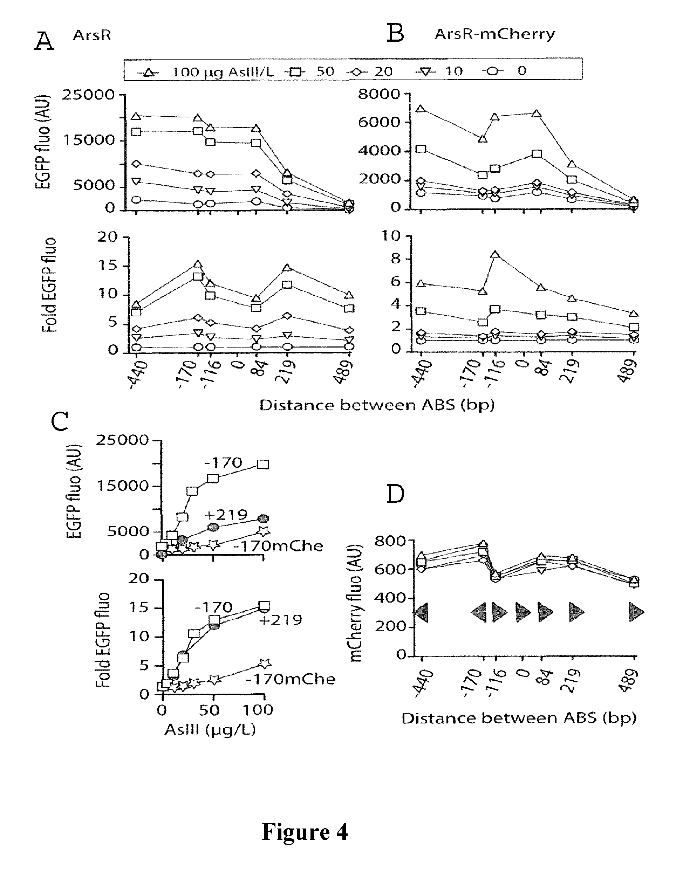

**Figure 4** compares up- and downstream positioning of the second ArsR operator (ABS) within the *arsR-egfp* circuitry. (A) EGFP fluorescence and fold-change as a function of distance between the two ABS, after 2 h induction time with different arsenite (AsIII) concentrations as measured by flow cytometry. (B) as (A), but for constructs expressing ArsR-mCherry. (C) EGFP fluorescence and fold-change as a function of arsenite concentration for clarity, for three different circuitries (relative distance between ABS indicated). (D) ArsR-mCherry fluorescence as a function of distance between the ABS, at different arsenite concentrations. Dark grey triangles represent the direction of the second ABS relative to the ABS within the Pₐᵣₛ-promoter.

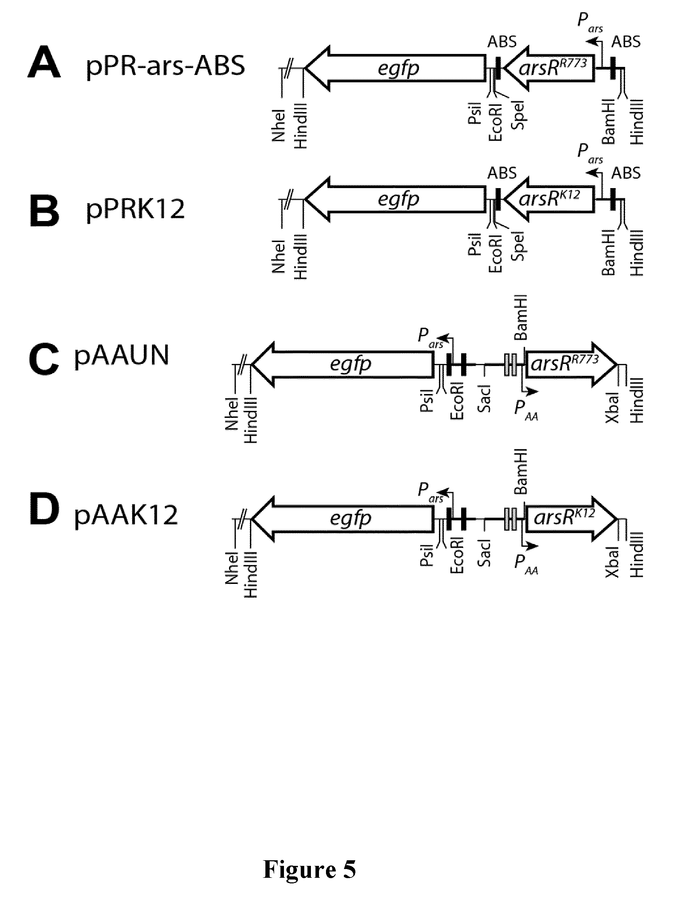

**Figure 5** represents an overview of different reporter plasmids. (A) and (B) Feedback constructs carrying *arsR^{R773}* or *arsR^{K12},* which express both *arsR* and *egfp* from the upstream P_{ars,R773} promoter. (C) and (D) Uncoupled constructs in which expression of *arsR* is driven by the constitutive P_{AA}-promoter, but expression of *egfp* is maintained under P_{ars, R773}. Black vertical lines indicate the ArsR operator (from the original P_{ars,R773} promoter).

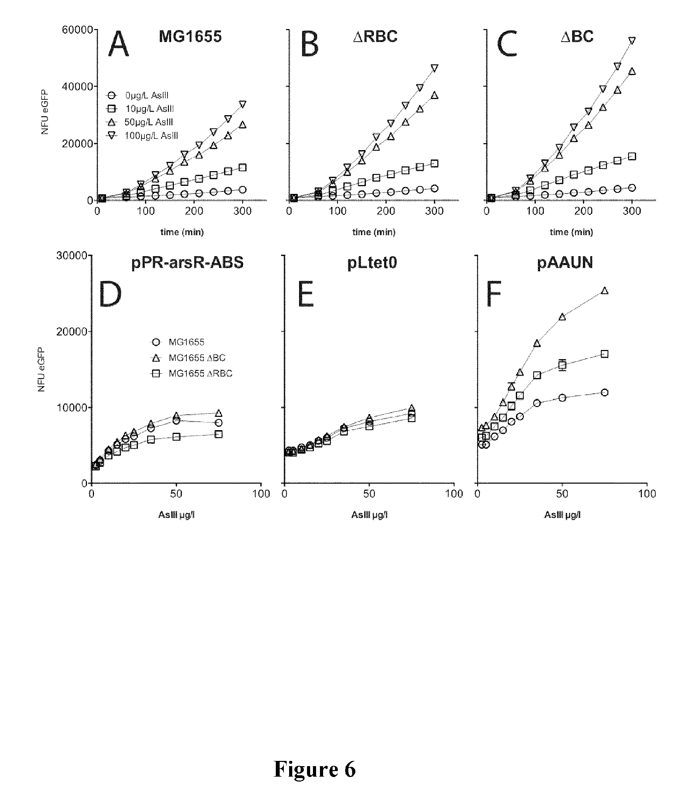

**Figure 6** shows EGFP induction of ArSR^{R773} based bioreporters in different *E*. *coli* K12 backgrounds, measured by fluorometry. (A)-(C) Induction kinetics over time of EGFP expression from pPR-arsR-ABS in *E*. *coli* MG1655, MG1655 ΔRBC and MG1655 ΔBC, respectively. (D)-(F) EGFP response versus arsenite (AsIII) concentration (after 160 min induction) in the three *E*. *coli* hosts for pPR-ars-ABS (D) versus the uncoupled circuits pTetL0 (E) and pAAUN (F). Note that pTetL0 is a similar reporter circuit in which the P_{AA} promoter is replaced by the stronger P_{LTet0} promoter. Data points are culture turbidity normalized averages from triplicate spectrofluorometer measurements. Errors bars are calculated standard deviations from the average. When not visible, the error bars are smaller than the used symbol size.

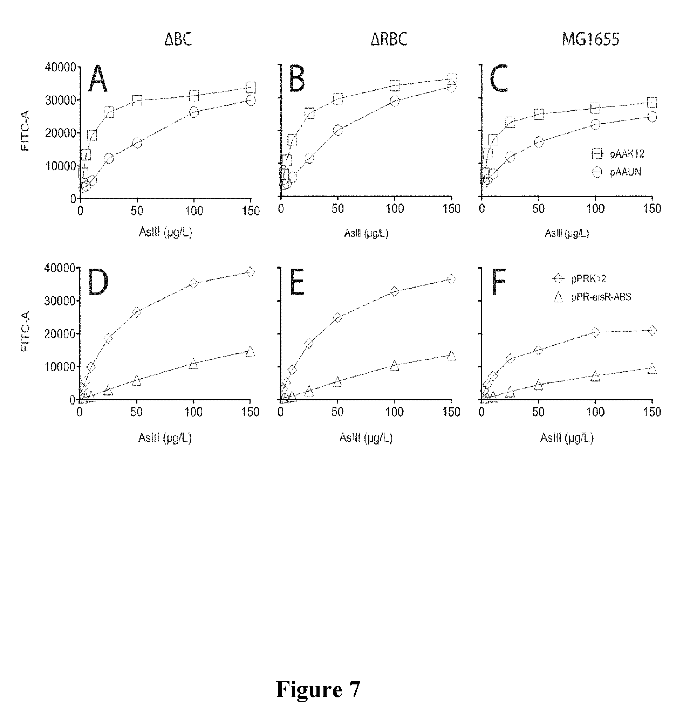

**Figure 7** shows EGFP expression as a function of arsenite (AsIII) exposure in different *E. coli* hosts and for either ArsR^{R773} or ArsR^{K12} based bioreporters, as measured by flow cytometry. (A) *E*. *coli* MG1655 ΔBC pAAK12 (open squares) versus pAAUN (open circles) after 180 min of induction. (B) and (C) *E*. *coli* MG1655 ΔBC and *E*. *coli* MG1655, respectively. (D)-(F) *E*. *coli* with pPRK12 (open diamonds) or pPR-arsR-ABS (open triangles). Dots represent averages from triplicate measurements, standard deviation whiskers are smaller than the used symbols. FITC-A, values from the channel to measure the EGFP fluorescence.

### Detailed Description of the invention

### Definitions

The term "pollutant" as used herewith encompasses inorganic and organic toxic compounds generally found in water such as drinking water. It includes, for instance, arsenic ions (arsenite (As(III)), arsenate (As(V))), antimonite (Sb(III)), cadmium (Cd²⁺), zinc (Zn²⁺), mercury (Hg²⁺), silver (Ag²⁺), bismuth (Bi³⁺), borate (BO₃³⁻), cobalt (Co²⁺), chromate (CrO₄²⁻), copper (Cu²⁺), nickel (Ni²⁺), lead (Pb³⁺), antimony (Sb²⁺), tellurate (TeO₃²⁻), thallium (Tl⁺) ions.

As used herewith "an operon allowing detoxification of a pollutant in bacteria" refers to the system of defence against toxic compound(s) and/or metabolism (in particular catabolism) of toxic compound(s) to render said compound(s) inactive, which certain microorganisms like bacteria have naturally developed. The "operon" designates a functioning unit of genomic DNA containing a cluster of genes under the control of a single promoter. The genes are transcribed together into an mRNA strand and either translated together in the cytoplasm, or undergo trans-splicing to create monocistronic mRNAs that are translated separately, i.e. several strands of mRNA that each encode a single gene product. The result of this is that the genes contained in the operon are either expressed together or not at all. Control of an operon is a type of gene regulation that enables organisms to regulate the expression of various genes depending on environmental conditions. Operon regulation can be either negative or positive by induction or repression. In negative inducible operons like *ars* operon, a regulatory repressor protein (e.g. ArsR) is normally bound to the operator (ArsR binding site, ABS), which prevents the transcription of the genes on the operon. If an inducer molecule is present (e.g. a toxic compound like arsenite), it binds to the repressor and changes its conformation so that it is unable to bind to the operator. This allows for expression of the operon.

As used herewith, the terms "liquid samples" cover water samples including drinking water, environmental water (e.g. water from rivers, groundwater), urine or blood, water extracts from food stuffs and soils.

As used herewith, the terms "derive" or "derivative" applied to a protein or to a nucleic acid refer to an amino acid sequence or nucleic acid sequence that is substantially homologous to the referenced sequence from which it derives, but which has an amino acid sequence or nucleic acid sequence different from that of the referenced sequence because of one or more amino acid or one or more nucleotide deletions, insertions and/or substitutions. A substantially homologous amino acid sequence can be at least 80%, at least 85%, at least 90%, at least 95%, at least 96%, at least 97%, at least 98% or at least 99% identical to the referenced amino acid sequence. A substantially homologous nucleic acid sequence can be at least 80%, at least 85%, at least 90%, at least 95%, at least 96%, at least 97%, at least 98% or at least 99% identical to the referenced nucleic acid sequence. The identity of two amino acid sequences or of two nucleic acid sequences can be determined by visual inspection and/or mathematical calculation, or more easily by comparing sequence information using known computer program used for sequence comparison such as Clustal package version 1.83. A derivative may comprise a sequence having at least one conservatively substituted amino acid, meaning that a given amino acid residue is replaced by a residue having similar physiochemical characteristics.

### Products according to the invention

A first aspect of the invention relates to a biologically based test system for the detection of inorganic and organic pollutants in liquid samples, such as water samples and in particular drinking water samples, comprising a genetically modified bacterium producing a reporter protein upon induction of a regulatory protein in presence of said pollutant, wherein the nucleic acid(s) of said bacterium comprises:
(a) a first transcription unit comprising (i) a nucleic acid encoding a reporter protein placed under the control of the promoter from an operon allowing detoxification of said pollutant in bacteria wherein said pollutant is capable of interacting with a regulatory protein of said operon, (ii) a first DNA binding site for said regulatory protein located upstream from said promoter, and (iii) at least a second DNA binding site for said regulatory protein located upstream or downstream from said promoter, and
(b) a second transcription unit comprising (i) a nucleic acid encoding said regulatory protein placed under the control of a constitutive promoter.

As one skilled in the art will understand from current application, the different aspects of the invention are applicable to any system allowing detoxification of pollutants involving a regulatory protein that regulates the expression of at least one gene having detoxification effects and/or catabolic effects upon induction in the presence of a specific pollutant as for instance the systems of detoxification of arsenic ions (arsenite (As(III)), arsenate (As(V))), antimonite (Sb(III)), cadmium (Cd²⁺), zinc (Zn²⁺), mercury (Hg²⁺). Other pollutants which can be detected with the invention include, for instance, silver (Ag²⁺), bismuth (Bi³⁺), borate (BO₃³⁻), cobalt (Co²⁺), chromate (CrO₄²⁻), copper (Cu²⁺), nickel (Ni²⁺), lead (Pb³⁺), antimony (Sb²⁺), tellurate (TeO₃²⁻), thallium (Tl⁺).

In particular, is provided a biologically based test system for the detection of inorganic pollutants selected from the group consisting of arsenite, arsenate and antimonite in liquid samples including water samples, wherein the nucleic acid(s) of said bacterium comprises:
(a) a first transcription unit comprising (i) a nucleic acid encoding a reporter protein placed under the control of the promoter from an *ars* operon, (ii) a first DNA binding site for an ArsR regulatory protein located upstream from said promoter, and (iii) at least a second DNA binding site for said ArsR regulatory protein located upstream or downstream from said promoter, and
(b) a second transcriptional unit comprising (i) a nucleic acid encoding said ArsR regulatory protein placed under the control of a constitutive promoter.

In one embodiment, the biologically based test system according to the invention comprises a genetically modified bacterium as defined above comprising two DNA binding sites for said regulatory protein.

In a particular embodiment, the first DNA binding site for said regulatory protein is located upstream from said promoter, at a distance from said promoter that is identical to the distance found between the DNA binding site and the promoter of said operon in a bacteria having naturally developed said operon.

In another embodiment, the second DNA binding site for said regulatory protein is located upstream from said promoter and upstream from the first DNA binding site.

In a still other embodiment, the second DNA binding site for said regulatory protein is located downstream from said promoter and downstream from the first DNA binding site.

According to the invention, the distance between two DNA binding sites for said regulatory protein is comprised between a minimum distance avoiding mutual steric interactions between said bound regulatory protein and a maximum distance still allowing synergistic effects of increasing concentrations of said regulatory protein, caused by the local interaction of said regulatory protein with the binding site. Said minimum and maximum distances can be evaluated by placing the two DNA binding sites at known distances on the chromosome of a bacterium expressing a fluorescently labelled ArsR functional derivative and by measuring the intensity of the fluorescent gradient as a function of the relative positioning of the two DNA binding sites, using standard methods in the field such as by single cell epifluorescence microscopy, for instance as described by Hammar et al. (Science, 2012, 336:1595-1598*).*

In a particular embodiment, the distance between two DNA binding sites for said regulatory protein is comprised between about 80 bp and about 500 bp, more particularly between about 80 bp and about 490 bp, more particularly between about 80 bp and about 450 bp, more particularly between about 80 and about 250 bp, still more particularly between about 80 and 220 bp, or between about 80 and 200 bp or between about 100 and 180 bp, or between about 110 and 180 bp, or between about 115 and about 170 bp, or between about 150 and about 220 bp.

In a particular embodiment, in the first transcription unit, the first DNA binding site is located upstream from said promoter, the second DNA binding site is located downstream from said promoter, and the distance between the two DNA binding sites is comprised between about 80 and 250 bp, in particular between about 80 and 220 bp, more particularly between about 84 or 85 bp and 219 or 220 bp.

In a still particular embodiment, in the first transcription unit, the first DNA binding site is located upstream from said promoter, the second DNA binding site is located downstream from said promoter, and the distance between the two DNA binding sites is about 85 bp, e.g. of 80, 81, 82, 83, 84, 85, 86, 87, 88, 89, or 90 bp.

In a still particular embodiment, in the first transcription unit, the first DNA binding site is located upstream from said promoter, the second DNA binding site is located downstream from said promoter, and the distance between the two DNA binding sites is about 220 bp, e.g. of 215, 216, 217, 218, 219, 220, 221, 222, 223, 224, or 225 bp.

In another particular embodiment, in the first transcription unit, both the first and the second DNA binding sites are located upstream from said promoter, and the distance between the two DNA binding sites is comprised between about 80 and 200 bp, in particular between about 100 and 180 bp, or more particularly between about 115 or 116 bp and 170 bp.

In a still other particular embodiment, in the first transcription unit, both the first and the second DNA binding sites are located upstream from said promoter, and the distance between the two DNA binding sites is about 170 bp, e.g 165, 166, 167, 168, 169, 170, 171, 172, 173, 174 or 175 bp.

In a still other particular embodiment, in the first transcription unit, both the first and the second DNA binding sites are located upstream from said promoter, and the distance between the two DNA binding sites is about 115 bp, e.g 110, 111, 112, 113, 114, 115, 116, 117, 118, 119, 120 or 121 bp.

As mentioned in the background section, in bacteria, resistance to arsenic ions and antimony are associated with plasmid-encoded resistance operons (e.g. *arsRDABC* operon on *E. coli* plasmid R773), which are widespread among bacterial species, although many bacteria carry arsenite resistance genes on the chromosome (e.g. *arsRBC* operon in *E. coli* K12 or MG1655).

In one embodiment of the invention, the nucleic acid encoding said ArsR regulatory protein comprised in the second transcription unit can derive from the naturally occurring *E. coli* plasmid R773 or from *E. coli* chromosomal *ars* operon.

In a specific embodiment, the nucleic acid encoding said ArsR regulatory protein comprised in the second transcription unit derives from the naturally occurring *E. coli* plasmid R773, in particular the nucleic acid encoding said ArsR regulatory protein has at least 80%, at least 90%, at least 95%, at least 96%, at least 97%, at least 98%, at least 99%, or 100% identity with the nucleic acid sequence of the allele of the *arsR* gene naturally present in *E. coli* plasmid R773 of SEQ ID NO: 1.

In another embodiment of the expression vector according to the invention, the nucleic acid encoding said ArsR regulatory protein comprised in the second transcription unit has a nucleic acid sequence deriving from the sequence of the allele of the *arsR* gene naturally present on the chromosome of a bacterium, in particular the nucleic acid encoding said ArsR regulatory protein has at least 80%, at least 90%, at least 95%, at least 96%, at least 97%, at least 98%, at least 99%, or 100% identity with the nucleic acid sequence of the allele of the *arsR* gene naturally present in the chromosome of *E. coli* K12 of SEQ ID NO: 2.

In one additional specific embodiment, the nucleic acid encoding said ArsR regulatory protein in the second transcription unit and the promoter in the first transcription unit derive from the same plasmidic *ars* operon or from the same chromosomal *ars* operon, e.g. from the same plasmidic *ars* operon from the *E. coli* plasmid R773 or from the same chromosomomal *ars* operon of *E. coli* such as *E. coli* K12.

In a still specific embodiment, the nucleic acid encoding said ArsR regulatory protein in the second transcription unit and the promoter in the first transcription unit derive from the chromosomal *ars* operon from *E. coli* such as *E. coli* K12.

More specifically, the nucleic acid encoding said ArsR regulatory protein in the second transcription unit has the nucleic acid sequence of SEQ ID NO: 2 and the promoter in the first transcription has the nucleic acid sequence of SEQ ID NO: 3.

In one embodiment of the invention, said first and second transcription units are located on the chromosome or on a bacterial plasmid of the genetically modified bacterium.

In a particular embodiment of the invention, the genetically modified bacterium is an *Escherichia coli* strain bearing a genetically modified plasmid, said plasmid carrying:
(a) a first transcription unit comprising (i) a nucleic acid encoding a reporter protein placed under the control of the promoter from an *ars* operon, (ii) a first DNA binding site for an ArsR regulatory protein located upstream from said promoter, and (iii) a second DNA binding site for said ArsR regulatory protein located upstream or downstream from said promoter, and
(b) a second transcriptional unit comprising (i) a nucleic acid encoding said ArsR regulatory protein placed under the control of a constitutive promoter.

In a further particular embodiment of the invention, the genetically modified bacterium is an *E. coli* strain, in particular *E. coli* K12 or a strain derived from strain K12 such as *E. coli* MG1655 *(*Riley et al, 2006, Nucleic Acids Res 34: 1-9*),* optionally deleted for at least one of chromosomal *arsB, arsC* and *arsR* genes.

In a still other particular embodiment of the invention, the genetically modified bacterium is *E. coli* MG1655 deleted for chromosomal *arsB* and *arsC* genes and, optionally, also deleted for chromosomal *arsR* gene.

In one embodiment of the invention, the nucleic acid encoding a reporter protein is a nucleic acid encoding a chromogenic label (e.g. horseradish peroxidase, alkaline phosphatase, β-galactosidase) or fluorescent compound (e.g. Green Fluorescent Protein and derivatives thereof including enhanced green fluorescent protein (EGFP), Red Fluorescent Protein, Yellow Fluorescent protein and derivatives thereof including venus), or luminescent labels including luciferins.

Examples of constitutive promoters comprised in the second transcriptional unit useful in the invention include a "moderately" constitutive promoter such as P_{AA}-promoter or a "strong" constitutive promoter such as P_{Ltet0} promoter described in Alper et al (PNAS, 2005, 102: 12678-12683*).*

According to the invention, the biologically based test system can comprise a genetically modified bacterium as described herewith, which is in the form of a whole cell without disruption of its cell wall or in the form of cell-free lysate. Both forms are appropriate for the use of the biologically based test system for the detection of inorganic and organic pollutants in liquid samples.

In another aspect, it is provided a recombinant expression vector for use in a biologically based test system for the detection of inorganic and organic pollutants in liquid samples such as water samples comprising:
(a) a first transcription unit comprising (i) a nucleic acid encoding a reporter protein placed under the control of the promoter from an operon allowing detoxification of said pollutant in bacteria wherein said pollutant is capable of interacting with a regulatory protein of said operon, (ii) a first DNA binding site for said regulatory protein located upstream from said promoter, and (iii) a second DNA binding site for said regulatory protein located upstream or downstream from said promoter, and
(b) a second transcription unit comprising (i) a nucleic acid encoding said regulatory protein placed under the control of a constitutive promoter.

The nucleotide sequences mentioned above can be inserted in the recombinant expression vector by methods well known to a person skilled in the art such as, for example, those that are described in MOLECULAR CLONING: A LABORATORY MANUAL, Sambrook et al., 4th Ed., Cold Spring Harbor Laboratory Press, Cold Spring Harbor, N.Y., 2001.

The first and second transcription units comprised in the recombinant expression vector of the invention are generally independently regulated.

As mentioned above, the different aspects of the invention are applicable to any system allowing detoxification of pollutants involving a regulatory protein that regulates the expression of at least one gene having detoxification effects upon induction in the presence of a specific pollutant as for instance the systems of detoxification of arsenic (arsenite (As(III)), arsenate (As(V))), antimonite (Sb(III)), cadmium (Cd²⁺), zinc (Zn²⁺), mercury (Hg²⁺), silver (Ag²⁺), bismuth (Bi³⁺), borate (BO₃³⁻), cobalt (Co²⁺), chromate (CrO₄²⁻), copper (Cu²⁺), nickel (Ni²⁺), lead (Pb³⁺), antimony (Sb²⁺), tellurate (TeO₃²⁻), thallium (Tl⁺) ions.

In a particular embodiment, is provided a recombinant expression vector for use in a biologically based test system for the detection of inorganic pollutants selected from the group consisting of arsenite, arsenate and antimonite in liquid samples such as water samples comprising:
(a) a first transcription unit comprising (i) a nucleic acid encoding a reporter protein placed under the control of the promoter of the *ars* operon, (ii) a first DNA binding site for an ArsR regulatory protein located upstream from said promoter, and (iii) a second DNA binding site for an ArsR regulatory protein located upstream or downstream from said promoter, and
(b) a second transcriptional unit comprising (i) a nucleic acid encoding said ArsR regulatory protein placed under the control of a constitutive promoter.

In general, the expression vector according to the invention further comprises a gene conferring resistance to an antibiotic such as kanamycin and/or ampicillin for maintaining a selection pressure in bacteria transformed with such vectors in order not to lose said vector after multiple successive divisions of the bacteria. For that purpose, the bacteria are cultivated in presence of the antibiotic against which the vector confers a resistance to the bacteria.

Generally, said expression vector can be any plasmid that can be maintained and expressed in a bacterium like *E. coli.*

In one embodiment of the invention, said expression vector is a derivative of the *E. coli* K12 plasmids pBR322, pUC18, pACYC184 or RSF1010.

One skilled in the art will understand that the same particular embodiments detailed above for the different elements of the first and second transcription units comprised in the biologically test system according to the invention also apply to the recombinant expression vectors according to the invention.

In a particular embodiment, the expression vectors according to the invention are selected from the group consisting of: pAA-2ABS+489, pAA-2ABS+219, pAA-2ABS+84, pAA-2ABS-440, pAA-2ABS-170 and pAA-2ABS-116, as described in the Example section and Figure 2 (where pAAUN corresponds to pAA-2ABS+84), or derive from those plasmids.

The names of the vectors according to the invention recapitulate the main features distinguishing the vectors between each other's and provide, from left to right, the following indications: the first three letters identify the promoter type controlling the transcription of the *arsR* gene (e.g. pAA), the second group of five characters (e.g. - 2ABS) refers to the number of ABS present in total in the system and the last group of digits beginning with a "+" or a "-" (e.g. +489) defines the distance between the first ABS (in the natural positioning relative to the Pars promoter controlling the transcription of the reporter gene) and the second ABS, this number is positive when the second ABS is placed downstream from the Pars promoter and negative when the second ABS is placed upstream from the Pars promoter.

In a more particular embodiment, the expression vectors according to the invention are selected from the group consisting of: pAA-2ABS-440, pAA-2ABS-170 and pAA-2ABS-116, as described in the Example section and Figure 2, or derive from those plasmids.

Thus, in a particular embodiment, the expression vector according to the invention comprises:
(a) a first transcription unit comprising (i) a nucleic acid encoding a reporter protein placed under the control of the promoter of the *ars* operon, (ii) a first DNA binding site for an ArsR regulatory protein located upstream from said promoter, and (iii) a second DNA binding site for an ArsR regulatory protein located upstream from said promoter at a distance of about 116 bp, about 170 bp, or about 440 bp from said first DNA binding site, and
(b) a second transcriptional unit comprising (i) a nucleic acid encoding said ArsR regulatory protein placed under the control of the pAA promoter.

In a further particular embodiment, the expression vector according to the invention comprises:
(a) a first transcription unit comprising (i) a nucleic acid encoding a reporter protein placed under the control of the promoter of the *ars* operon, (ii) a first DNA binding site for an ArsR regulatory protein located upstream from said promoter, and (iii) a second DNA binding site for an ArsR regulatory protein located upstream from said promoter at a distance of about 116 bp or about 170 bp from said first DNA binding site, and
(b) a second transcriptional unit comprising (i) a nucleic acid encoding said ArsR regulatory protein placed under the control of the pAA promoter.

In another particular embodiment, the expression vector according to the invention comprises:
(a) a first transcription unit comprising (i) a nucleic acid encoding a reporter protein placed under the control of the promoter of the *ars* operon, (ii) a first DNA binding site for an ArsR regulatory protein located upstream from said promoter, and (iii) a second DNA binding site for an ArsR regulatory protein located downstream from said promoter at a distance of about 84 bp, about 219 bp, or about 489 bp from said first DNA binding site, and
(b) a second transcriptional unit comprising (i) a nucleic acid encoding said ArsR regulatory protein placed under the control of the pAA promoter.

All the vectors according to the invention illustrated in the example section are responsive to arsenite concentrations lower than 10 µg/l, they differ for the level of background noise (signal in absence of inducer) and overall signal production eventually leading to a broad range of signal to noise ratio.

In a more particular embodiment, the expression vector according to the invention is pAA-2ABS-170 as described in the Example section and figure 2A, or derives from this plasmid.

The expression vector pAA-2ABS-170, or a derivative thereof, comprises:
(a) a first transcription unit comprising (i) a nucleic acid encoding a reporter protein placed under the control of the promoter of the *ars* operon, (ii) a first DNA binding site for an ArsR regulatory protein located upstream from said promoter, and (iii) a second DNA binding site for an ArsR regulatory protein located upstream from said promoter at a distance of about 170 bp from said first DNA binding site, and
(b) a second transcriptional unit comprising (i) a nucleic acid encoding said ArsR regulatory protein placed under the control of the pAA promoter.

The expression vector pAA-2ABS-170 is particularly useful for the purpose of arsenite detection in water samples since it presents both low background noise and high overall signal, as demonstrated in the Example section, and thus allows for precise readout by means of lower quality reader.

In a still other particular embodiment, the expression vector according to the invention is pAA-2ABS-116 as described in the Example section and figure 2A, or derives from this plasmid.

The expression vector pAA-2ABS-116, or a derivative thereof, comprises:
(a) a first transcription unit comprising (i) a nucleic acid encoding a reporter protein placed under the control of the promoter of the *ars* operon, (ii) a first DNA binding site for an ArsR regulatory protein located upstream from said promoter, and (iii) a second DNA binding site for an ArsR regulatory protein located upstream from said promoter at a distance of about 116 bp from said first DNA binding site, and
(b) a second transcriptional unit comprising (i) a nucleic acid encoding said ArsR regulatory protein placed under the control of the pAA promoter.

In an alternative embodiment are provided expression vectors as specifically described above, wherein the pAA promoter of the second transcriptional unit under (b) is replaced by any other constitutive promoter.

In a further aspect of the invention is provided a host cell comprising a recombinant expression vector as described herewith.

In a still further aspect is provided an isolated genetically modified bacterium, for use in a biologically based test system for the detection of inorganic and organic pollutants in liquid samples such as water samples, producing a reporter protein upon induction of a regulatory protein in presence of said pollutant, wherein the nucleic acid(s) of said bacterium comprises:
(a) a first transcription unit comprising (i) a nucleic acid encoding a reporter protein placed under the control of the promoter from an operon allowing detoxification of said pollutant in bacteria wherein said pollutant is capable of interacting with a regulatory protein of said operon, (ii) a first DNA binding site for said regulatory protein located upstream from said promoter, and (iii) at least a second DNA binding site for said regulatory protein located upstream or downstream from said promoter, and
(b) a second transcription unit comprising (i) a nucleic acid encoding said regulatory protein placed under the control of a constitutive promoter.

As already mentioned, the different aspects of the invention are applicable to any system allowing detoxification of pollutants involving a regulatory protein that regulates the expression of at least one gene having detoxification effects upon induction in the presence of a specific pollutant.

Thus, in a particular embodiment, the isolated genetically modified bacterium is as described above, wherein the pollutant is selected from the group consisting of arsenite, arsenate and antimonite, said operon is an *ars* operon, and said regulatory protein is the ArsR regulatory protein.

As will be understood by the skilled person, the isolated genetically modified bacterium according to the invention can further have one or more of the above mentioned particular features, such as those related to the relative positioning of the two DNA binding sites and/or the distance between said DNA binding sites, those related to the genetic background of said bacterium such as the strain from which it derives, the deletion of at least one chromosomal *arsB* and *arsC* genes and optionally the deletion of chromosomal *arsR* gene, the allele of the nucleic acid encoding the ArsR regulatory protein, and those related to the reporter protein.

In a particular embodiment, the isolated genetically modified bacterium according to the invention is as described above, wherein, in said first transcription unit, the first and second DNA binding sites are located upstream from said promoter.

In another particular embodiment, the isolated genetically modified bacterium according to the invention is as described above, wherein the distance between two DNA binding sites is comprised between about 80 bp and about 500 bp, in particular between about 80 bp and about 250 bp, more particularly between about 80 and 220 bp or 80 and 200 bp, and/or between about 100 and 180 bp, in particular between about 110 and 180 bp, or more particularly between about 115 and 170 bp.

In a particular embodiment, the isolated genetically modified bacterium according to the invention is an *E. coli* strain, in particular *E. coli* MG1655, deleted for at least one chromosomal *arsB, arsC* and/or *arsR* gene.

In another particular embodiment of the isolated genetically modified bacterium according to the invention, the nucleic acid encoding said ArsR regulatory protein in the second transcription unit has the nucleic acid sequence of SEQ ID NO: 2 and the promoter in the first transcription has the nucleic acid sequence of SEQ ID NO: 3.

In another aspect is provided a biologically based test kit for the detection of inorganic and organic pollutants in liquid samples such as water samples, in particular for the detection of inorganic pollutants selected from the group consisting of arsenite, arsenate and antimonite, comprising genetically modified bacteria according to the invention producing a reporter protein upon induction of a regulatory protein in presence of a pollutant.

In one embodiment of the kit according to the invention, said bacteria have been encapsulated in agarose microbeads of a diameter ranging from 40-70 µm.

In another embodiment of the kit according to the invention, said bacteria have been lyophilized.

Typically, for use in the detection of the pollutant, said lyophilized bacteria according to the invention are reconstituted with the liquid sample to be tested.

In a further aspect is provided a device for the detection of inorganic and organic pollutants in liquid samples such as water samples, in particular for the detection of inorganic pollutants selected from the group consisting of arsenite, arsenate and antimonite, comprising genetically modified bacteria according to the invention.

In a particular embodiment, the device according to the invention comprises lyophylized genetically modified bacteria according to the invention and two gas-permeable plastic sheets casted together forming multiple cavities containing said bacteria.

In a further particular embodiment, said bacteria have been lyophilized and are sealed into multiple cavities of, for instance, about 200 µl of volume, formed by casting two gas-permeable plastic sheets together.

In said embodiment, the water sample can be injected, with the help of a syringe, through a rubber septum directly into a channel that leads to the cavities comprising the genetically modified bacteria of the invention.

For example, 24 spots of lyophilised whole cell genetically modified bacteria according to the invention can be sealed in cavities obtained by die-casting of two plastic sheets. Additionally, channels connect triplicate spots to the same rubber septum and the sample can be injected with a syringe needle.

In a particular embodiment, the device according to the invention is a portable device. In the kit and devices according to the invention, the genetically modified bacteria can be in the form of whole cells without disruption of their cell wall or in the form of cell-free lysate.

After usage, the kits and devices according to the invention can be disposed easily by autoclaving or incineration.

The kit and device according to the invention can be stored at -20°C or at -80°C for several weeks.

Another aspect of the invention relates to a biologically based test system for the detection of inorganic and organic pollutants in liquid samples such as water samples, comprising a genetically modified bacterium producing a reporter protein upon induction of a regulatory protein in presence of said pollutant, wherein the nucleic acid(s) of said bacterium comprises:
(a) a nucleic acid comprising (i) a nucleic acid encoding a reporter protein placed under the control of the promoter from an operon allowing detoxification of said pollutant in bacteria wherein said pollutant is capable of interacting with a regulatory protein of said operon, in particular the promoter of the chromosomal form of said operon (ii) a first DNA binding site for said regulatory protein located upstream from said promoter, and (iii) at least a second DNA binding site for said regulatory protein located upstream or downstream from said promoter, and
(b) a nucleic acid identical to the chromosomal nucleic acid encoding said regulatory protein.

In a particular embodiment, the nucleic acid encoding said regulatory protein under (b) is placed under the control of a constitutive promoter in a transcription unit independent of the transcription unit comprising the nucleic acid under (a).

As will be understood by the skilled person, the biologically based test system according to the invention can further be characterized by one or more of the above mentioned particular features, such as those related to the relative positioning of the two DNA binding sites and/or the distance between said DNA binding sites, those related to the genetic background of said bacterium such as the strain from which it derives, the deletion of at least one chromosomal *arsB* and *arsC* genes and optionally the deletion of chromosomal *arsR* gene, and those related to the reporter protein.

A particular embodiment of the invention relates to a biologically based test system for the detection of inorganic pollutants selected from the group consisting of arsenite, arsenate and antimonite, in liquid samples such as water samples, comprising a genetically modified bacterium producing a reporter protein upon induction of an ArsR regulatory protein in presence of said pollutant, wherein the nucleic acid(s) of said bacterium comprises:
(a) a nucleic acid comprising (i) a nucleic acid encoding a reporter protein placed under the control of the promoter of *ars* operon, in particular the promoter of chromosomal *ars* operon of *E. coli* K12 of SEQ ID NO: 3, (ii) a first DNA binding site for said ArsR regulatory protein located upstream from said promoter, and (iii) at least a second DNA binding site for said regulatory protein located upstream or downstream from said promoter, and
(b) a nucleic acid identical to the chromosomal nucleic acid encoding said ArsR regulatory protein in a bacterium, such as a nucleic acid of SEQ ID NO: 2.

In a particular embodiment, the nucleic acid encoding said ArsR regulatory protein under (b) is placed under the control of a constitutive promoter in a transcription unit independent of the transcription unit comprising the nucleic acid under (a).

In a particular embodiment, the nucleic acid encoding said ArsR regulatory protein under (b) is placed under the control of the promoter of the *ars* operon in the same transcription unit as the transcription unit comprising the nucleic acid under (a).

In another particular embodiment, in the nucleic acid under (a), the first and second DNA binding sites are located upstream from said promoter.

In the two above embodiments, the test system of the invention is particularly useful for detecting arsenite, arsenate or antimonite present at concentrations ranging from near 0 to about 150 µg/l, more particularly below or equal to 50 µg/l, still more particularly below or equal to 10 µg/l or between about 10 and about 50 µg/l, in liquid samples including water samples.

In another particular embodiment of the biologically based system according to the invention, the distance between two DNA binding sites for said regulatory protein is comprised between about 80 bp and about 500 bp, in particular between about 80 bp and about 250 bp, more particularly between about 80 and 220 bp or 80 and 200 bp, and/or between about 100 and 180 bp, in particular between about 110 and 180 bp, or more particularly between about 115 and 170 bp.

### Methods and uses according to the invention

One aspect of the invention relates to a method for preparing and/or isolating a genetically modified bacterium producing a reporter protein upon induction of a regulatory protein in presence of a pollutant as described herewith.

Said method of preparation comprises introducing the recombinant expression vector according to the invention in a host cell such as a bacterium. The introduction of the recombinant expression vector in a host cell can be carried out according to methods that are well known to a person skilled in the art, such as those described in BASIC METHODS IN MOLECULAR BIOLOGY, Davis et al., 2nd ed., McGraw-Hill Professional Publishing, 1995, and MOLECULAR CLONING: A LABORATORY MANUAL, supra, such as transfection by calcium phosphate, transfection by DEAE dextran, transfection, microinjection, transfection by cationic lipids, electroporation, transduction or infection.

In another aspect is provided a method for preparing a biologically based test kit according to the invention comprising the steps of:
(a) cultivating genetically modified bacteria according to the invention in an appropriate culture medium,
(b) removing said culture medium,
(c) lyophilizing the bacteria.

Standard methods in the field for cultivating micro-organisms and for lyophilizing (freeze-drying) microorganisms can be used. Typically, in the freeze-drying process, bacteria are suspended in a suitable protective medium, frozen and exposed to a vacuum to remove water from the frozen sample. After drying, the bacteria are typically stored under vacuum in glass vials.

Another aspect of the invention relates to a method for preparing a device for the detection of inorganic and organic pollutants in liquid samples such as water samples, in particular for the detection of inorganic pollutants selected from the group consisting of arsenite, arsenate and antimonite, comprising:
(a) cultivating genetically modified bacteria according to the invention in an appropriate culture medium,
(b) removing said culture medium,
(c) lyophilizing the bacteria,
(d) placing said lyophilized bacteria in the multiple cavities formed between two sealed gas-permeable plastic sheets.

A still other aspect of the invention relates to the use of a biologically based test system, kit or device according to the invention for the detection of inorganic and organic pollutants, in particular for the detection of inorganic pollutants selected from the group consisting of arsenite, arsenate and antimonite, in liquid samples such as water samples. A still other aspect of the invention relates to the use of a biologically based test system, kit or device as defined above, wherein the nucleic acid(s) of said bacterium comprises:
(a) a first transcription unit comprising (i) a nucleic acid encoding a reporter protein placed under the control of the promoter of *ars* operon, in particular the promoter of chromosomal *ars* operon of *E. coli* K12 of SEQ ID NO: 3, (ii) a first DNA binding site for said ArsR regulatory protein located upstream from said promoter, and (iii) at least a second DNA binding site for said regulatory protein located upstream or downstream from said promoter, and
(b) a second transcription unit comprising a nucleic acid encoding said ArsR regulatory protein of SEQ ID NO: 2 placed under the control of a constitutive promoter, wherein said second transcription unit under (b) is independent of the first transcription unit under (a),
for detecting arsenite, arsenate or antimonite present at concentrations equal to or lower than about 50 µg/l, in particular equal to or lower that about 10 µg/l, in liquid samples such as water samples.

A still other aspect of the invention relates to the use of a biologically based test system, kit or device as described above, wherein the nucleic acid(s) of said bacterium comprises:
(a) a nucleic acid comprising (i) a nucleic acid encoding a reporter protein placed under the control of the promoter of *ars* operon, in particular the promoter of chromosomal *ars* operon of *E. coli* K12 of SEQ ID NO: 3, (ii) a first DNA binding site for said ArsR regulatory protein located upstream from said promoter, and (iii) at least a second DNA binding site for said regulatory protein located upstream or downstream from said promoter, and
(b) a nucleic acid encoding said ArsR regulatory protein of SEQ ID NO: 2 placed under the control of the promoter of the *ars* operon in the same transcription unit as the transcription unit comprising the nucleic acid under (a),
for detecting arsenite, arsenate or antimonite present at concentrations comprised between about 5 and 150 µg/l, in liquid samples such as water samples.

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the appended claims.

The invention having been described, the following examples are presented by way of illustration, and not limitation.

### Examples

### Materials and methods for examples 1 to 3

### Strains, plasmids and culture conditions

All vectors described herewith have been developed from pAAUN and pAAUN-mChe originally presented in Merulla et al (microbial Biotechnology, 2013, 5:503-514*).* All strains are based on *E. coli* MG1655 ΔRBC, a strain in which the chromosomal *arsRBC-*operon is deleted. *E. coli* strains were cultured on LB medium (Sambrook et al, Molecular Cloning: A Laboratory Manual. Third Edition ed. 2000*)* at 37°C with inclusion of the appropriate antibiotics to maintain the plasmid reporter constructs, except for bioreporter assays (see below).

### ABS designs in homologous setting

The starting design consisted of an "uncoupled" variant of the natural Pₐᵣₛ*-arsR* feedback system, in which *arsR* is placed under control of the constitutive P_{AA}-promoter and is divergently oriented to the *egfp* gene, which is under control of the Pₐᵣₛ-promoter (*Merulla et al, 2013, supra*). Three variants were produced, in which an extra copy of the ABS was placed upstream of *egfp* but at increasing distances downstream of the transcription start site (Figure 2). The sequence used for the spacer region consisted of a frameshift *arsR* gene sequence, so as to be able to compare ABS-distance effects between the variants having two ABS versus the ones having only a single ABS. Hereto, a 576-bp region consisting of the ABS sequence, followed by Pₐᵣₛ and *arsR,* and again an ABS sequence as in (Stocker et al., 2003, Environ Sci Technol 37:4743-4750) was *de novo* synthesized (DNA2.0, Inc.), but with addition of a 5' SacI and a 3' PsiI site, and with an extra C-nucleotide at position 30 to introduce a frame shift mutation in *arsR* (ϕ*arsR*). This results in stop codons at position 31, 46, 103 and 256. Furthermore, positions 156 and 426 of ϕ*arsR* were mutated to create two KpnI sites. By replacing the SacI-PsiI fragment of the previously described plasmid pAAUN (*Merulla et al, 2013, supra*) with the newly synthesized piece, plasmid pAA-2ABS+489 was produced, in which the distance between the two ABS was 489 bp (Figure 2). Next, by removing the 270-bp KpnI-KpnI fragment and self-ligation, pAA-2ABS+219 (distance between the ABSs 219 bp) was produced.

Variants carrying a single ABS were then produced from pAAUN (also called pAA-2ABS+84 according to the nomenclature described herewith), pAA-2ABS+489, and pAA-2ABS+219 by EcoRI digestion and recircularization, which removes a 81-bp fragment containing the ABS proximal to *egfp* (resulting in plasmids pAA-1ABS+67, pAA-1ABS+425, pAA-1ABS+155, respectively (Figure 2).

Next, a set of plasmids were created in which the second ABS is located at varying distance upstream of the Pₐᵣₛ promoter (Figure 2). First, by using primers of SEQ ID NO: 4 and SEQ ID NO: 5 in the polymerase chain reaction (PCR), the ϕ*arsR-*ABS fragment was amplified while adding a SacI restriction site at its 3'-end. After gel extraction and purification the fragment was inserted via TA cloning in pGEM-T-Easy (Promega). The orientation and sequence of the insert were verified. The part containing the ϕ*arsR*-ABS fragment was retrieved by digestion with SacI and cloned in the SacI site of pAA-1ABS+67. Depending on the orientation of the insert this produced plasmids pAA-2ABS-116 and pAA-2ABS-440 (Figure 2). Finally, by removing by KpnI digestion the internal fragment from ϕ*arsR* in pAA-2ABS-440, plasmid pAA-2ABS-170 was produced (Figure 2). All procedures were subsequently repeated starting from plasmid pAAUN-mChe, which carries an *arsR-mCherry* fusion gene and enables measurement of the ArsR-mCherry levels in the cells (*Merulla et al, 2013, supra*). All constructs were validated by sequencing.

### Design of reporter circuits with ABS in heterologous environment

To test whether the ArsR-ABS system could control a heterologous inducible promoter, the HbpR/P_{C}-promoter from *P. azelaica* HBP1 was used. As starting point and native control, *E. coli* (pHBP269) was used, which carries both the *hbpR* gene and, divergently oriented, the *egfp* gene under control of the P_{C}-promoter. The ABS was inserted downstream of the P_{C}-promoter on pHBP269 by recovering an 81-bp EcoRI-EcoRI ABS fragment from pPR-arsR-ABS (*Stocker et al, 2003, supra*), and inserting this in both orientations into the unique EcoRI site on pHBP269 (Figure 2). After transformation into *E. coli* this resulted in plasmid pHBP-ABS-dir1, in which the ABS is in the native orientation with respect to the direction of the Pc- (and Pₐᵣₛ) promoter, and pHBP-ABS-dir2, in which the ABS is in the opposite direction. To exert ArsR control, the *E. coli* strains were additionally transformed with plasmid pGEM-pAA-ArsR that contains a PCR amplified P_{AA}-*arsR* fragment from pAAUN using primers of SEQ ID NO: 6 and of SEQ ID NO: 7. In this plasmid *arsR* is under the control of the P_{AA} promoter and further downstream of the endogenous P_{lac} promoter of pGEM-T-Easy.

### Bioreporter assays

Starting from a single colony, *E. coli* bioreporter strains were grown for 16 h at 37°C and 160 rpm agitation of the culture flask in LB medium in the presence of 50 µg/ml kanamycin (Km) to select for the presence of the pAAUN- or pHBP-based reporter plasmids and, when required, 100 µg/ml ampicillin (Ap) to select for pGEM-pAA-ArsR. The bacterial culture was then 100-fold diluted into fresh LB medium plus Km and incubated for two hours at 160 rpm agitation until the culture turbidity at 600 nm had reached between 0.3 and 0.4 (representative for exponentially growing cells).

Cells from 10 ml culture were then harvested by centrifugation at 4,000 ×g for 5 min and at room temperature. The cell pellet was resuspended into 30°C preheated MOPS medium to a final optical density at 600 nm of 0.1 (MOPS medium contains 10% [v/v] of MOPS buffer, 2 mM MgCl₂, 0.1 mM CaCl₂, 2 g glucose per liter, and is set at pH 7.0). MOPS buffer itself was prepared by dissolving, per liter: 5 g NaCl, 10 g NH₄Cl, 98.4 g 3-([N-morpholino]propanesulfonic acid, sodium salt), 0.59 g Na₂HPO₄·2H₂O, and 0.45 g KH₂PO₄. This cell suspension was subsequently used for the reporter assays.

### Bioreporter assay preparation and readout.

Bioreporter assays were prepared in 96 well microplates (Greiner bio-one). In the case of single induction with arsenic an aliquot of 180 µl of bioreporter cell suspension in MOPS was mixed with 20 µl aqueous solution containing between 0 and 1 mg arsenite (AsIII) per liter, prepared by serial dilution of a 0.05 M solution of NaAsO₂ (Merck) in arsenic-free tap water. In the case of induction with 2-hydroxybiphenyl (2HBP), stock solutions were made from 0 to 10 mM of 2HBP in pure dimethylsulfoxide (DMSO), from which working solutions were prepared by dilution of 1:100 in arsenic-free tap water. As negative control, the same dilution of DMSO in water was added. Reporter assays with double induction were prepared by mixing 160 µl of MOPS bioreporter cell suspension with 20 µl of 2HBP working solution and 20 µl of arsenite aqueous solution. Bioreporter assays were prepared in triplicate and incubated at 30°C under mixing at 500 rpm for 3 h in the case of single arsenic induction assay and 2 h for the double induction in a 96-well thermostated shaker (THERMOstar, BMG Labtech). After the indicated incubation times 5 µl of each assay was removed, twice diluted by mixing with 195 µl of distilled water (final dilution 1:10,000), after which 3 µl was aspired and immediately analyzed on a Becton Dickinson LSR-Fortessa flow cytometer (BD Biosciences, Erembodegem, Belgium). mCherry fluorescence of individual cells was collected in the 'Texas-Red' channel (610/20 nm), whereas EGFP-fluorescence was measured using the 'FITC' channel (530/30 nm).

### Example 1. A secondary ArsR binding site reduces background expression but retains induction from Pars

A series of *arsR*-Pₐᵣₛ constructs without feedback loop was created with either a single ABS (within Pₐᵣₛ) or with an additional ABS further downstream from Pₐᵣₛ but upstream of the *egfp* reporter gene (Figure 2). For proper comparison between constructs with a single or with double ABS, the length and the DNA sequence of the 5'UTR of the *egfp* mRNA was maintained as closely as possible (Figure 2). The same DNA as in the native *arsR* feedback loop was chosen but this *arsR* gene was inactivated by frameshifts causing premature translational stops.

In the construct with single ABS within Pₐᵣₛ and the longest 5'UTR of *egfp* (plasmid pAA-1ABS+425), EGFP was only twofold induced at 100 µg As/l, as a result of high background expression in the absence of arsenite (Figure 3A, B). Successively shortening the distance between the Pₐᵣₛ-promoter and *egfp* slightly improved the induction potential of the system, but the background expression remained high (Figure 3A, B).

In contrast, constructs with the same length *5'egfp* UTRs that carry an additional ABS further downstream of Pₐᵣₛ but directly upstream of *egfp,* displayed a comparatively much lower background signal (Figure 3A, grey data points). EGFP fluorescence also diminished with increasing length of the spacer region and at the same arsenite concentration, but, importantly, the fold induction of the signal remains much higher compared to constructs having one ABS only (Figure 3B). Shortening the spacer between the two ABS copies improved both absolute EGFP signal intensity and fold induction, from 10-fold at 489 bp distance to 15-fold at 219 bp and at 100 µg As/l (Figure 3B). At shorter ABS distances (e.g., in pAA-2ABS+67) the absolute EGFP intensity increased further, but the induction level decreased (Figure 3B). Reduction of background EGFP expression is thus not an effect of the 5'UTR sequence or its length, but must be due to the additional ABS and its interactions with ArsR.

*Conclusion:* showing that the increase of the 5'UTR length only partially explains the decrease of overall signal produced by the addition of a secondary ABS confirms that the ArsR-ABS can control the transcription rate via a mechanism independent from the transcription initiation process.

### Example 2. Decreased background expression from upstream placed ArsR binding sites

It was tested whether placing an additional ABS upstream of the native ABS in the Pₐᵣₛ-promoter would also affect expression of the reporter gene. Reporter plasmids with an additional ABS placed at 116, 170 and 440 bp upstream of Pₐᵣₛ were constructed as described above (Figure 2B). Surprisingly, upstream-placed ABS also reduced background EGFP reporter expression in absence of arsenite, almost to the level of placing the second ABS immediately downstream of the transcriptional start site (Figure 4A). On the contrary, the EGFP fluorescence levels upon addition of arsenite remained very high (Figure 4A). When considering the fold induction, the upstream placed secondary ABSs were, therefore, as good as the downstream placed variants (Figure 4B). Across all distances of the second ABS compared to the original ABS in Pₐᵣₛ, optimal signal-to-noise ratios (up to 15-fold induction at 50 µg As/l) occurred at relative distances of 170-219 bp up- or down-stream, diminishing to 6-fold at shorter and longer relative distances (Figure 4B). Since the absolute EGFP fluorescence signal is more than twofold higher at the same arsenite concentration from plasmid pAA-2ABS-170 than from pAA-2ABS+219, placing a second ABS upstream is actually more favorable for signal detection (Figure 4C). On the other hand, if low background signal in itself is an issue, then the variants with the second ABS downstream show more efficient reduction (Figure 4A).

As effects of ABS placement on *egfp* expression may be masked by varying ArsR levels in the different constructs, expression of ArsR from P_{AA} was examined. Similar plasmid constructs as above were used except that *arsR* was replaced with an *arsR-mCherry* translational fusion (Figure 2B). Constructs producing ArsR-mCherry instead of ArsR showed similar but slightly different arsenite-dependent EGFP expression (Figure 4B). Notably, EGFP fluorescence levels in *arsR-mCherry* strains were threefold lower than in *arsR* strains, and -fold inductions were less pronounced (Figure 4B).

In contrast to the two induction optima in the *arsR* strains (at -170 and +219 bp distance of the second ABS), the *arsR-mCherry* strains showed a single optimum around -116 bp (Figure 4D). Measurement of mCherry fluorescence confirmed that expression of ArsR-mCherry from P_{AA} is insensitive to the presence or not of arsenite. Expression of ArsR-mCherry from P_{AA} varied slightly as a function of the relative distance and position of both ABSs around Pₐᵣₛ (Figure 4D), but this is not sufficient to explain the large effect of secondary ABS placement on *egfp* expression (Figure 4B). Therefore, effects of ABS placement within the global environment of the Pₐᵣₛ promoter are due to ArsR interacting with the ABS, and not to fluctuating levels of ArsR itself.

*Conclusion:* these results showed that adding a second ABS at a favorable distance upstream Pars leads to a decrease of the transcriptional noise without affecting the overall transcriptional rate and eventually increasing the fold induction.

### Example 3. Heterologous application of the ArsR transcriptional roadblock

To test whether the ArsR-ABS system can be used in a heterologous promoter context (i.e., non*-arsR* origin), circuits in which *egfp* reporter gene expression is controlled by the HbpR activator protein (Jaspers et al., 2001, Microbiology, 147:2183-94*)* were constructed. HbpR is a σ⁵⁴-RNA polymerase dependent transcription factor, which activates the P_{C}-promoter upstream of the *hbpC* gene in the presence of the effector 2-hydroxybiphenyl (2HBP).

EGFP fluorescence was about 35-fold higher after 2 h incubation with 10 µM 2HBP in *E. coli* carrying plasmids pHBP269 (without ABS) and pGEM-pAA-arsR, a plasmid in which ArsR is constitutively expressed from the P_{AA}-promoter. The results showed that EGFP expression from the P_{C}-promoter on pHBP269 was not influenced by the presence of sub-toxic amounts of arsenite between 5 and 100 µg/l. In contrast, inclusion of the ABS sequence downstream of the P_{C}-promoter but upstream of *egfp* as in pHBP-ABS-dirl made the reporter circuit dependent on ArsR, and reduced EGFP expression fourfold in absence of arsenite. Addition of arsenite to those cells relieved ArsR control in an arsenite-concentration dependent manner, with 2HBP induction being almost completely restored to the level without ABS at 100 µg AsIII/l. Placing the ABS in the opposite direction relative to the promoter (as in pHBP-ABS-dir2) caused very little effect and only slight repression by ArsR occurred.

It was confirmed by Northern hybridization that the control exerted by the added heterologous ABS occurred at the transcriptional level.

*Conclusion:* this showed that ArsR-ABS can control expression of a reporter gene in a heterologous promoter context given that the orientation of the ABS sequence relative to the direction of transcription is like in the natural conformation.

### Materials and Methods for Examples 4 to 6

### Chromosomal ars deletions in E. coli

Deletion of the complete *arsRBC* operon from *E. coli* MG1655 was described in *Merulla et al (2013, supra*). The same procedure was repeated to remove only the *arsBC* genes while leaving *arsR* intact (producing *E. coli* MG1655 ΔBC).

### pAAK12 constriction

The *arsR*^{K12} gene was amplified from the genome of *E. coli* MG1655 by PCR, while adding the same ribosome binding site upstream of *arsR*^{R773} and two flanking restriction sites (BamHI and XbaI). Chromosomal DNA was prepared from a freshly grown colony *of E.* coli MG1655 (16 h) on solid LB media, which was resuspended in 100 µl of double distilled water and boiled for 5 min. After centrifugation at 10,000 x g for 1 min 1 µl of the supernatant was recovered and used as template in the PCR. The reaction was carried out with Taq-polymerase (Promega) at a Tm of 55°C, with elongation steps of 40 s, and 25 cycles, in presence of primer of SEQ ID NO: 8 (carrying a BamHI site and the *arsR*^{R773} ribosome binding site) and primer of SEQ ID NO: 9 (carrying an XbaI site). The PCR fragment was purified from agarose gel and ligated to prepared pGEM-T easy vector (Promega). After transformation into *E. coli* this resulted in plasmid pGEM_arsR-K12. The *arsR-K12* insert was validated by DNA sequencing.

pAAK12 was then assembled by recovering the *arsR* fragment from pGEM_arsR_K12 by XbaI-BamHI digestion, which was ligated with the Pₐᵣₛ-*egfp* fragment from pAAUN (BamHI-NheI digested) and the linearized vector pPROBE (cut with NheI-XbaI). The final plasmid was validated for the correct composition of the *arsR*^{K12}*-*Pₐᵣₛ*-egfp* fragment. Note that the Pₐᵣₛ promoter is of R773 origin. The *arsR* ^{K12} gene was expressed from the constitutive P_{AA} promoter and is oriented in the opposite direction from Pₐᵣₛ.

### pPRK12 constriction

To replace the *arsR* allele in the feedback reporter circuit of pPR-arsR-ABS (i.e., *arsR* under control of Pₐᵣₛ), a newly designed DNA sequence was synthesized (DNA2.0) of SEQ ID NO: 10.

This fragment was recovered from the production plasmid by digestion with PsiI and SalI, and ligated with the vector part of pPR-arsR-ABS cut with the same enzymes. After transformation into *E. coli* and verification of the plasmid content, this resulted in plasmid pArs-arsRK12-ABS.

### Microfluidic cartridge fabrication

The microfluidic cartridge was composed of a polydimethylsiloxane (PDMS) block containing the designed channels and cages bonded on a typical microscopy glass slide with thickness of 1 mm. The PDMS block was fabricated from a silicon mold, which contained the negative imprint of the cages and channels. To fabricate the silicon mold, a silicon wafer was structured by dry etching (Bosch process, Alcatel 601E) using a photolithographically structured AZ1512 resist (Clariant) as a mask. Designs were made using Clewin (WieWeb) and transferred to a chrome mask by laser writing. Then, using this mask, they were transferred to the positive photoresist by selective UV exposure.

To fabricate the PDMS block, 10 g of Sylgard 184 silicone elastomer base (Dow Coming) was mixed with 1 g of Sylgard 184 silicone elastomer curing agent (ratio 10:1). Then, PDMS was degassed under vacuum for 20 min to remove air bubbles, poured on the silicon mold and baked for 2 h at 80°C to become solid and taking the shape of the mold. Afterwards, the PDMS was peeled off. In order to seal the channels, the PDMS block was irreversibly bonded onto a 1 mm thick glass slide by treating the surfaces with oxygen plasma (0.6 mbar, 100 W, 1 min, Diener Electronic - Femto) and placing the imprinted PDMS surface onto the glass. One silicon mold served for several dozen PDMS blocks. On each glass slide 4 chips, with a total of 16 flow lines, were bonded.

### Arsenic induction assay in microfluidic device

Appropriate dilutions of a 50 mM stock solution of sodium arsenite (NaAs02, Merck) or of a 13 mM stock solution of sodium arsenate were prepared in (arsenic free) tap water to obtain a 1 mg/l arsenite working solution. Whole cell bioreporter suspensions were mixed immediately before loading into the microfluidics cartridge with solutions containing final arsenite concentrations of between 2.5 and 200 µg/l, with tap water alone as negative control. Seven µl of this bioreporter-arsenic suspension was loaded into an inlet of 12 lines in a PDMS cartridge. Inlets were subsequently connected to a custom made volumetric pump via a short 1.06 mm diameter PTFE tube (Fisher Scientific). The filling and connection procedure took around five min, after which the cartridge was pressurized and the bioreporter cells started to flow and accumulate in the filter cage. EGFP fluorescence from the cells accumulated on the filter was followed by digital microscope imaging on a Leica inverted microscope at 200-fold magnification over a time period up to 3 h at 20 °C. Exposure times were set to 66 ms with a gain factor of 10 and the lowest intensity of the light source. Reporter signals were quantified by digital image analysis using MetaMorph Offline version 7 Or3 (Molecular Device) graphic analysis software, and are expressed as arbitrary units of image intensity (A.U.)

### Arsenic bioreporters assays

Arsenic reporter assays for fluorimetry were performed as described in *Merulla et al (2013, supra*). In other cases, flow cytometry was used to measure the expression of EGFP in the reporter cells. This was done following the procedure detailed for examples 1 to 3.

### Example 4. Different ars alleles occurring in E. coli arsenic bioreporter strains

Plasmid R773 has been described because of its ability to confer to *E. coli* resistance to high levels of arsenate (AsV), arsenite (AsIII) and arsenic trioxide, although it also confers resistance to tetracycline and streptomycin. On this plasmid, a single 4.4 kb operon (named *arsRDABC* for the five genes it comprises) is responsible for the production of the proteins of the detoxification system and for regulation of its own expression. Genes for arsenic resistance are also frequently found on the chromosome of prokaryotic species. In *E. coli* MG1655 the chromosomal *ars* operon (i.e. *arsRBC*) differs from that of R773 in lacking *arsD* and *arsA.*

Blastn and Blastp comparison between *arsR* from plasmid R773 (*arsR*^{R773}, Genbank accession number P15905, SEQ ID NO: 1) indicated 74% nucleotide and 77% amino acid identity to the chromosomal version of *E. coli* K12 *(arsR*^{K12}*,* Genbank accession number AAC76526, SEQ ID NO: 2). In contrast, *arsR*^{R773} shows 91% nucleotide identity to ArsR from the chromosome of *Salmonella enterica* (Genbank accession number NP_863361.1.), suggesting its origin may have been *Salmonella enterica.*

*E. coli* strains that are used as hosts for arsenic bioreporter circuits may thus potentially show and suffer from crosstalk (i.e. interaction between one element of one system and one element of the other system) between the two ArsR alleles for control of Pars mediated transcription. This would occur when ArsR^{R773} would bind the ars^{K12}-promoter, or ArsR^{K12} the P_{arsR773} operator, or when ArsR^{R773}-ArsR^{K12} would form heterodimers. In addition, deletion of the genes for the ArsB pump could make cells reacting more sensitively to lower arsenite concentration in the extracellular medium. Indeed, *E. coli* MG1655 strains without the chromosomal *ars* operon divided much slowly than wild-type in the presence of arsenite, but this was only evident at arsenite concentrations of 19 mg/l and beyond (not shown).

### Example 5. Effect of ars chromosomal operon on the expression of the reporter gene from the plasmid located Pₐᵣₛ-promoter

It was first tested if and how deletion of the chromosomal *ars* operon would affect expression of a reporter gene controlled by ArsR^{R773} and P_{ars R773} in presence or absence of arsenic. Two types of deletions were made. In the first, the *arsB* and *arsC* genes were deleted, but *arsR^{K12}* (and its promoter and suspected operator site) was left intact. In the second, the complete *arsRBC* chromosomal region was deleted (while leaving the promoter and suspected operator).

Host strains were transformed in first instance with the regular feedback circuit on pPR-arsR-ABS, in which case *egfp* is expressed from ArsR^{R773} and P_{ars,R773} (Figure 5). EGFP induction could be observed by fluorimetry after 60 and 90 min of exposition to arsenite. The level of normalized EGFP fluorescence (i.e., corrected by culture turbidity) at the same arsenite concentration was higher in *E. coli* with chromosomal *ars* deletions than in *E. coli* wild-type MG1655 (Figure 6). The increase was highest in *E*. *coli* MG1655 ΔRB as host, with about 1.5-times increase in normalized EGFP fluorescence at 100 µg As/liter. Deletion of the chromosomal *ars* operon had no effect on induction of the same strains with arsenate.

In comparison to the feedback reporter circuit, the uncoupled circuit could magnify the effect of the chromosomal *ars* deletion (Figure 6D-F), but this was dependent on the strength of the promoter used to produce *arsR.* For example, in pAAUN *arsR^{R773}* expression was driven from the "moderately" constitutive P_{AA}-promoter but in pLTet0UN from the stronger P_{Ltet0} promoter (*Merulla et al, 2013, supra*). Deletion of *arsRBC* and even more so of *arsBC* drastically increased the EGFP expression from pAAUN, but this was almost undetectable in case of pLTet0UN (Figure 6E, F).

Since fluorometry is not ideal for reporter measurements because of the necessity to control for culture turbidity, and because of potential interference with fluorescence from the culture medium itself, induction assays were repeated in microfluidic systems, in which the EGFP fluorescence from the cells is detected by epifluorescence microscopy and quantitative image analysis. Induction upon addition of arsenite to the reference circuit, *E. coli* MG1655 (pPR-ars-ABS), led to increase of the EGFP signal over time, and as a function of arsenite concentration (not shown). The EGFP signal was quite low, though, but induction of *E. coli* MG1655 with the uncoupled arsenic reporter (pAAUN) led to a much stronger signal development. Expression of EGFP from pAAUN also increased in *E. coli* with chromosomal *arsR* deletions, increasing to about 1.5-fold at 100 µg As/l in MG1655 ΔRBC and to 2-fold in MG1655 ΔBC. Whereas the total EGFP signal increase in pAAUN constructs, however, the background expression also increased.

*Conclusion:* These results show that deleting the chromosomal *ars* operon (or at least *arsB* and *arsC* genes) from the chromosome of *E. coli* MG1655 increased the responsivity of both the coupled and uncoupled reporter circuits. Furthermore, it was clear that the saturation level of EGFP expression seen in hosts without chromosomal *ars* operon was lower than in the host only deleted for *arsB* and *arsC,* suggesting that the concomitant expression of the two ArsR alleles affects the repression efficiency of the ArsR-ABS system.

### Example 6. Effect of homogenous versus heterogenous reporter-host background

It was tested whether replacing *arsR*^{R773} with *arsR*^{K12} in the bioreporter circuit itself would change the inducibility of the reporter gene as a function of arsenite in the various host backgrounds. Two variants were produced, one in which *arsR* in the feedback circuit was replaced (pPRK12) and the other in which *arsR* was replaced in the uncoupled circuit (pAAK12) (Figure 5). For this comparison, flow cytometry was used for quantification of EGFP expression, since it has no measurable background and allows superb single-cell analysis.

Most strikingly, for both circuit types and in all genetic backgrounds, replacement of *arsR^{R773}* by *arsR^{K12}* led to a strong accentuation of the exponential character of the induction curve: a stronger response up to about 50 µg AsIII/l, then to reach an approximately similar saturation level of expression at higher arsenite concentrations (Figure 7). Deletion of the chromosomal *ars* operon also invariably led to between 1.2-and 2-fold higher EGFP signal values, as observed in previous Example. Varying the allele in the reporter circuit caused noticeable effects primarily below 25 µg AsIII/l, where the K12 allele responded with a much higher response than the R773 allele. In particular for the feedback-based bioreporters, replacing *arsR^{R773}* by *arsR^{K12}* led to a logarithmic (pPRK12) instead of a linear response for pPR-arsR-ABS between 0 and 150 µg As/l, and a much higher saturation level of EGFP expression (Figure 7D-F). Also in this case, it became evident that the basal EGFP expression was much lower in feedback than in uncoupled circuits, with pPR-arsR-ABS presenting 10 times less signal in absence of arsenite than pAAUN (almost irrespective of the absence or presence of chromosomal *ars*)*.*

As a consequence, the fold induction in the low range of arsenite concentrations (below 10 µg AsIII/l) is better for the *arsR*^{K12} variants, because of the increased steepness of the response curve (Figure 7A-C). In particular the pAAK12 circuit in *E*. *coli* MG1655 ΔBC responded with 2.5 to 3 fold-induction at 2.5 µg AsIII/l and with 6 to 8 folds at 10 µg AsIII/l (Table 1). In contrast, at higher concentration (150 µg/l AsIII) the feedback circuits performed better, with 22 to 38 folds for pPR-arsR-ABS and 15 to 23 folds for pPRK12 (Table 1).

**Table 1. Induction of EGFP expression for the different circuits and genetic backgrounds (calculated as the ratio between induced average signal (from triplicates) divided by the average basal expression)**

| AsII I µg/l | pAAUN | | | pAAK12 | | | pPR-arsR-ABS | | | pPRK12 | | |
|---|---|---|---|---|---|---|---|---|---|---|---|---|
| | ΔRBC | ΔBC | MG | ΔRBC | ΔBC | MG | ΔRBC | ΔBC | MG | ΔRBC | ΔBC | MG |
| 0 | 1 | 1 | 1 | 1 | 1 | 1 | 1 | 1 | 1 | 1 | 1 | 1 |
| 2.5 | 1.2 | 1.1 | 1.2 | 2.5 | 3.1 | 2.5 | 1.3 | 1.2 | 1.2 | 1.9 | 1.9 | 2 |
| 10 | 1.9 | 1.8 | 1.8 | 6.2 | 7.8 | 6.1 | 2.8 | 2.8 | 2.3 | 5.2 | 5.8 | 5.2 |
| 50 | 6.6 | 5.7 | 4.4 | 10.7 | 12.1 | 8.9 | 14.1 | 15.1 | 10.7 | 14.4 | 15.7 | 10.9 |
| 150 | 11 | 10 | 6.5 | 12.9 | 13.7 | 10.2 | 34.4 | 37.8 | 22.4 | 21.2 | 22.8 | 15.3 |

*Conclusion:* the incorporation of ArsR^{K12} offers new possibilities to tailor the reporter circuit in the concentration range up to 150 µg AsIII/l. As examples, the newly developed pAAK12 and pPRK12 bioreporter circuits could be extremely interesting: *E*. *coli* with pAAK12 had its sensitivity at 0-10 µg AsIII/l (after which it saturates), and *E*. *coli* pPRK12 mostly within 5-50 µg AsIII/l. In contrast, both pAAUN and pPR-arsR-ABS showed a more linear response range over the range of 0-150 µg AsIII/l. In addition, the increase in absolute signal intensity as in the *arsR^{K12}* sensors could be interesting when detection of the EGFP signal has to be performed by cheap low sensitivity detectors (rather than expensive instruments such as flow cytometers or epifluorescence microscopes).

In addition, the results obtained with microfluidic devices indicated that by combining the different concepts (e.g., uncoupled circuit, *arsBC* chromosomal deletion) one could sufficiently enhance signal response from the cells to enable detection by simple and cheap fluorescence detectors.

### Sequence listing:

nucleic acid sequence of the allele of the *arsR* gene naturally present in *E. coli* plasmid R773 : SEQ ID NO: 1 atgttgcaactaacaccacttcagttatttaaaaacctgtccgatgaaacccgtttgggtatcgtgttgttgctcaggga 
gatgggagagttgtgcgtgtgatctttgcatggcactggatcaatcacagcccaaaatatcccgtcatctggcgatgc
tacgggaaagtggaatccttctggatcgtaaacagggaaaatgggttcactaccgcttatcaccgcatattccttcatgg
gctgcccagattattgagcaggcctggttaagccaacaggacgacgttcaggtcatcgcacgcaagctggcttcagttaa
ctgctccggtagcagtaaggctgtctgcatctaa
nucleic acid sequence of the allele of the *arsR* gene naturally present in the chromosome of *E. coli* K12 : SEQ ID NO: 2 
atgtcatttctgttacccatccaattgttcaaaattcttgctgatgaaacccgtctgggcatcgttttactgctcagcga
actgggagagttatgcgtctgcgatctctgcactgctctcgaccagtcgcagcccaagatctcccgccacctggcattgc
tgcgtgaaagcgggctattgctggaccgcaagcaaggtaagtgggttcattaccgcttatcaccgcatattccagcatgg
gcggcgaaaattattgatgaggcctggcgatgtgaacaggaaaaggttcaggcgattgtccgcaacctggctcgacaaaa
ctgttccggggacagtaagaacatttgcagttaa
nucleic acid sequence of promoter of *arsR^{K12}*
   SEQ ID NO: 3:
   cagattaatcatatgcgtttttggttatgtgttgtttgacttaatatcagagccgagagatacttgttttctacaaagtaa
primers for amplification of ϕ*arsR*-ABS fragment
   SEQ ID NO: 4: GAGCTCTGTTGCAACTAACACCACTTCAG
   SEQ ID NO: 5: TCTTTAGTTAGTTAGGGAATTCACTAGTG
Primers for amplification of P_{AA}-*arsR* fragment from pAAUN
   SEQ ID NO: 6: AAA TCT GAG CTC CAA TTC CGA CGT CTA
   SEQ ID NO: 7: CTG CCA GGA ATT GGG GAT CGG AAG
primers for amplification of *arsR*^{K12} from *E*. *coli* MG1655
   SEQ ID NO: 8:
   cttGGATCCaaaggagaggggaaATGTCATTTCTGTTACCCATCCAATTG
   SEQ ID NO: 9: tctagaTTAACTGCAAATGTTCTTACTGTCCC
Synthetic DNA for pPRK12 construction : SEQ ID NO: 10

ggaagcttgcatgcctgcaggtcgactctagaggatccaagctttccaagttatctcacctaccttaaggtaatagtgtgattaat
catatgcgtttttggttatgtgttgtttgacttaatatcagagccgagagatacttgttttctacaaaggagagggaaatgtcatttct
gttacccatccaattgttcaaaattcttgctgatgaaacccgtctgggcatcgttttactgctcagcgaactgggagagttatgcgt
ctgcgatctctgcactgctctcgaccagtcgcagcccaagatctcccgccacctggcattgctgcgtgaaagcgggctattgct
ggaccgcaagcaaggtaagtgggttcattaccgcttatcaccgcatattccagcatgggcggcgaaaattattgatgaggcct
ggcgatgtgaacaggaaaaggttcaggcgattgtccgcaacctggctcgacaaaactgttccggggacagtaagaacatttg
cagttaaaaaatttgcctgaattccaagttatccacctaccttaaggtaatagtgtgattcatcatatgcgtttttggttatgtgaatta
atcactagtgaattccctaactaactaaagattaactttataagga.

## Claims

1. A biologically based test system for the detection of inorganic and organic pollutants in liquid samples comprising a genetically modified bacterium producing a reporter protein upon induction of a regulatory protein in presence of said pollutant, wherein the nucleic acid(s) of said bacterium comprises:
(a) a first transcription unit comprising (i) a nucleic acid encoding a reporter protein placed under the control of the promoter from an operon allowing detoxification of said pollutant in bacteria wherein said pollutant is capable of interacting with a regulatory protein of said operon, (ii) a first DNA binding site for said regulatory protein located upstream from said promoter, and (iii) at least a second DNA binding site for said regulatory protein located upstream from said promoter, and
(b) a second transcription unit comprising (i) a nucleic acid encoding said regulatory protein placed under the control of a constitutive promoter.

2. The biologically based test system according to claim 1, wherein the inorganic pollutant is selected from the group consisting of arsenite, arsenate and antimonite, and wherein said operon is an *ars* operon and said regulatory protein is ArsR.

3. The biologically based test system according to any one of claim 1 or 2, wherein said first transcription unit under (a) comprises two DNA binding sites for said regulatory protein and wherein the distance between two DNA binding sites for said regulatory protein is comprised between about 80 bp and about 500 bp.

4. The biologically based test system according to any one of claims 1 to 3, wherein, in said first transcription unit, the distance between said first and second DNA binding sites is comprised between about 100 and 180 bp.

5. The biologically based test system according to any one of claims 1 to 4, wherein, in said first transcription unit, both said first and said second DNA binding sites are located upstream from said promoter, and the distance between the two DNA binding sites is about 170 bp or about 115 or 116 bp.

6. The biologically based test system according to any one of claims 1 to 5, wherein the nucleic acid encoding said ArsR regulatory protein has at least 80% identity with SEQ ID NO: 2.

7. The biologically based test system according to any one of claims 1 to 6, wherein the nucleic acid encoding said ArsR regulatory protein in the second transcription unit comprises the nucleic acid sequence of SEQ ID NO: 2 and the promoter in the first transcription comprises the nucleic acid sequence of SEQ ID NO: 3.

8. The biologically based test system according to any one of claims 1 to 7, wherein the genetically modified bacterium is an *Escherichia coli* strain deleted for at least one of chromosomal *arsB, arsC* and *arsR* genes.

9. A recombinant expression vector for use in a biologically based test system for the detection of inorganic and organic pollutants in liquid samples such as water samples comprising:
(a) a first transcription unit comprising (i) a nucleic acid encoding a reporter protein placed under the control of the promoter from an operon allowing detoxification of said pollutant in bacteria wherein said pollutant is capable of interacting with a regulatory protein of said operon, (ii) a first DNA binding site for said regulatory protein located upstream from said promoter, and (iii) a second DNA binding site for said regulatory protein located upstream from said promoter, and
(b) a second transcription unit comprising (i) a nucleic acid encoding said regulatory protein placed under the control of a constitutive promoter.

10. The recombinant expression vector according to claim 9, wherein the inorganic pollutant is selected from the group consisting of arsenite, arsenate and antimonite, and wherein said operon is an *ars* operon and said regulatory protein is ArsR.

11. The recombinant expression vector according to any one of claim 9 or 10, wherein said first transcription unit under (a) comprises two DNA binding sites for said regulatory protein and wherein the distance between two DNA binding sites for said regulatory protein is comprised between about 80 bp and about 500 bp.

12. The recombinant expression vector according to any one of claims 9 to 11, wherein:
(a) in said first transcription unit, said second DNA binding site is located upstream from said promoter at a distance of about 115 or 116 bp, or about 170 bp from said first DNA binding site; and
(b) in said second transcription unit, said constitutive promoter is the pAA promoter.

13. An isolated genetically modified bacterium, for use in a biologically based test system for the detection of inorganic and organic pollutants in liquid samples such as water samples, producing a reporter protein upon induction of a regulatory protein in presence of said pollutant, wherein the nucleic acid(s) of said bacterium comprises:
(a) a first transcription unit comprising (i) a nucleic acid encoding a reporter protein placed under the control of the promoter from an operon allowing detoxification of said pollutant in bacteria wherein said pollutant is capable of interacting with a regulatory protein of said operon, (ii) a first DNA binding site for said regulatory protein located upstream from said promoter, and (iii) at least a second DNA binding site for said regulatory protein located upstream from said promoter, and
(b) a second transcription unit comprising (i) a nucleic acid encoding said regulatory protein placed under the control of a constitutive promoter.

14. A device for the detection of inorganic and organic pollutants in liquid samples comprising genetically modified bacteria according to claim 13.

15. Use of a biologically based test system according to any one of claims 1 to 8 for the detection of inorganic and organic pollutants in liquid samples such as water samples.
